# EUROPEAN PATENT APPLICATION

(11) **EP 2 514 759 A1**
(43) Date of publication of application: **24.10.2012**
(21) Application number: 10837633.6
(22) Date of filing: 15.12.2010
(51) Int. Cl.: C07J 63/00, A61K 31/08, A61K 31/085, A61K 31/12, A61K 31/135, A61K 31/136, A61K 31/185, A61K 31/19, A61K 31/56, A61K 31/704, A61P 1/16, A61P 31/14

(54) **TRITERPENE DERIVATIVE, AND PROPHYLACTIC OR THERAPEUTIC AGENT FOR CHRONIC HEPATITIS C**

(30) Priority: 15.12.2009 JP 2009284135
(71) Applicant: Meiji Seika Pharma Co., Ltd., Chuo-ku Tokyo (JP)
(72) Inventor: ISHIKAWA Makoto, Tokyo 104-8002 (JP); SUZUKI Shigeki, Tokyo 104-8002 (JP); AOKI Makoto, Yokohama-shi Kanagawa 222-8567 (JP); MINOWA Nobuto, Yokohama-shi Kanagawa 222-8567 (JP); SUZUKI Kokichi, Yokohama-shi Kanagawa 222-8567 (JP)
(74) Representative: Ziebell, Arnd
(86) International application number: PCT/JP2010/072564
(87) International publication number: WO 2011/074607

(57) **Abstract**

Provided are a triterpene derivative represented by a general formula (I), a pharmaceutically acceptable salt thereof, and an agent for preventing or treating chronic hepatitis C comprising the triterpene derivative or the salt as an active ingredient: 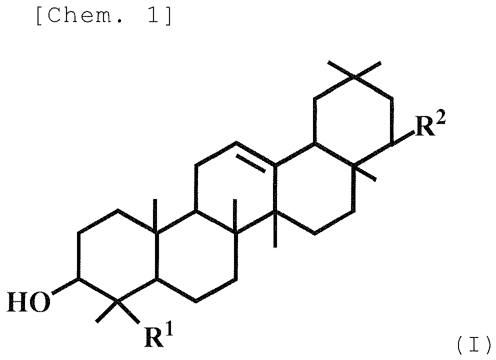 [in the formula (I), R¹ represents a carboxyl group, a hydroxymethyl group, -CH₂OSO₃H, or 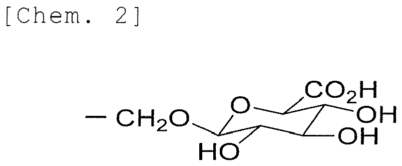 , and R² represents -OR³ or -O-(CH₂) m-OR⁴, where R³ represents a benzyl group which may be substituted with a hydroxymethyl group, a dimethylaminomethyl group, a phenylaminomethyl group, a morpholinomethyl group, a carboxyl group, or a formyl group, a C₁₋₆ alkyl group, a C₂₋₆ alkenyl group, a C₂₋₆ alkynyl group, or a hydroxy C₁₋₆ alkyl group, R⁴ represents a phenyl group which may be substituted with a carboxyl group, and m represents an integer of 1 to 3, with the proviso that a case where R¹ is -CH₂OH, and R³ is a C₁₋₆ alkyl group, a C₂₋₆ alkenyl group, or a benzyl group is excluded].

## Description

### [Technical Field]

The present invention relates to a novel triterpene derivative or a pharmaceutically acceptable salt thereof, and to an agent for preventing or treating chronic hepatitis C, the agent comprising the triterpene derivative or a pharmaceutically acceptable salt thereof as an active ingredient.

### [Background Art]

In Japan, an estimated 1.5 to 2.0 million people are persistently infected with hepatitis C virus (HCV), and it is assumed that most of them have chronic hepatitis C showing the symptoms of chronic hepatitis (see NPL 1). Based on observations of liver tissue, 80 to 90% of HCV-infected people have chronic hepatitis C, and there is a possibility that 50 to 60% of these infected people develop hepatic cirrhosis or hepatocellular carcinoma via chronic hepatitis (see NPL 2). Liver cancer is caused by HCV infection in many cases, and the number of deaths from liver cancer is approximately 30,000 per year (see NPL 3). Meanwhile, in other countries, although differing from country to country, an estimated 4.1 million people are infected with HCV (3.2 million people have chronic hepatitis) in the United States, and approximately 9.0 million people in Europe (see PTLs 4 and 5). Hepatitis C has become a major clinical problem worldwide.

A patient who is a subject of prevention or treatment of chronic hepatitis C is a patient infected with HCV. There are 6 major HCV genotypes from genotype 1 to genotype 6. It is known that particularly genotype 1 and genotype 2 are widespread worldwide (see NPLs 6 and 7). These genotypes further have subtypes, which can be distinguished from each other based on the base sequence at a specific region of RNA constituting the HCV, and the HCV load of each subtype can also be measured (see NPL 8).

In prevention or treatment of chronic hepatitis C, interferons (IFNs) are widely used to eliminate HCV. IFNs are bioactive substances produced by an innate immune system at the time of virus infection, and have an antiviral activity to inhibit viral replication. IFNs are species-specific proteins with a high degree of homology to each other. It is known that there are four classes of IFNs in humans (see NPL 9). At present, several different types of interferon α (IFN-α) and interferon β (IFN-β), including a type in which polyethylene glycol (PEG) is attached to an IFN for extending the time of being sustained in a body, are approved as pharmaceuticals exhibiting effects on chronic hepatitis C (see NPL 10).

It is known that the HCV load and the HCV genotype in the blood of a patient are important factors affecting the effects of IFNs on chronic hepatitis C. It has been revealed that the effects of the IFNs are low when the HCV load in the blood is a high viral load (1 Meq/ml, 5.0 Log IU/ml (100 KIU/ml) or 300 fmol/L or higher), or when the HCV genotype is genotype 1 or 4, and that the effects of the IFNs are the lowest particularly when the HCV genotype is 1b (see PTLs 11 and 12).

At present, a combination therapy of peginterferon (PEG-IFN) with ribavirin for 48 weeks is a standard therapy for a patient with chronic hepatitis C, and is expected as a therapy with the highest effect. However, the efficacy ratio is only approximately around 50% particularly for patients with HCV genotype 1b in the blood and having high viral loads who are the most difficult to treat (see NPL 11). This is considered to be mainly due to an influence of ribavirin that causes side effects such as anemia, besides the virus-side factors of genotype 1b exhibiting treatment resistance to the standard therapy, and other factors. These side effects necessitate the reduction in drug dose, and thus the therapeutic effect becomes insufficient, so that the treatment is discontinued or terminated (see NPLs 6 and 13). As described above, the existence of cases with refractory chronic hepatitis C that is difficult to treat with currently-available agents is a great medical issue to be addressed. In Japan, 70% or more of patients with chronic hepatitis C are patients with genotype 1b, and the percentage of elderly patients is also high.

Recently, researches have proceeded on drugs directly inhibiting HCV proliferation as therapeutic drugs for patients with chronic hepatitis C including cases with refractory chronic hepatitis C. These drugs are used in a three- to multiple-drug combination therapy by being added to the standard therapy using the combination of IFN with ribavirin. These drugs are expected to quickly remove HCV from bodies in cases including refractory chronic hepatitis C cases. However, problems yet to be solved are pointed out on the drugs directly inhibiting HCV proliferation, such as that the drugs induce HCV to have the drug resistance, and that the treatment cannot be completed by inherent side effects of the agents. At present, no drugs are available that can be generally used as an agent for preventing or treating cases with refractory chronic hepatitis C (see NPL 14). Additionally, to expect high therapeutic effects, combination with ribavirin is necessary, and the problem with ribavirin side effect is inevitable. If an agent useful for and highly safe in cases with refractory chronic hepatitis C is developed, these clinical problems will be solved. Such an agent is expected to be used in the future as an agent for treating a further refractory HCV subtype and the like, or as a therapeutic agent which can be used in multiple kinds of combinations with an agent directly inhibiting HCV proliferation, an anti-HCV drug containing an interferon preparation, or the like, and which has high therapeutic effects.

On the other hand, some triterpene derivatives are reported to have an effect of inhibiting hepatocellular injury (see PLTs 1 and 2). In particular, 22β-methoxyolean-12-ene-3β,24-diol, which is a triterpene derivative derived from soyasapogenol B, is reported not only to have the effect of inhibiting hepatocellular injury, but also to exhibit a synergistic anti-HCV activity when combined with interferon (see PTL 3).

However, anti-HCV activities of triterpene derivatives derived from soyasapogenol B other than 22β-methoxyolean-12-ene-3β,24-diol are not known.

### [Citation List]

### [Patent Literature]

[PTL 1] Japanese Patent No. 3279574
[PTL 2] Japanese Patent No. 3727353
[PTL 3] International Publication No. W02008/004653

### [Non Patent Literature]

[NPL 1] The Japan Society of Hepatology, ed., "Mansei Kanen No Chiryo Gaido (Guideline of Treatment for Chronic Hepatitis)", BUNKODO Co., Ltd., 2008, p. 21
[NPL 2] Iino Shiro, "C Gata Kanen Chiryo Gaido Rain (Guideline of Therapy for Hepatitis C", Nippon Rinsho, 2004, Vol. 62, Suppl. 7, p. 342-346
[NPL 3] Research Center for Cancer Prevention and Screening, National Cancer Center, Data on Deaths from Cancer based on Vital Statistics of Japan, (edited by Statistics and Information Dept., Minister's Secretariat, Ministry of Health, Labour and Welfare (from 1958 to 2005)
[NPL4] "Annals of Internal Medicine", (the United States), 2006, Vol. 144, p. 705-765
[NPL 5] "(ELPA, Summary expert recommendations)", (Belgium), 2009
[NPL 6] "New England Journal of Medicine", (the United States), 2001, Vol. 345, no. 1, p. 41-52
[NPL 7] "Journal of Clinical Microbiology", (the United States), 1994, Vol. 32, No. 4, p. 884-892
[NPL 8] "Journal of Clinical Microbiology", (the United States), 2006, p. 318-323
[NPL 9] "Annual Review of Biochemistry", (the United States), 1987, Vol. 56, p. 727-777
[NPL 10] [Mansei Kanen No Chiryo Gaido 2008 (Guideline of Treatment for Chronic Hepatitis)], edited by The Japan Society of Hepatology, 2008
[NPL 11] "B Gata C Gata Knnen Chiryo Ni Okeru Aratana Mondaiten (New problems in Hepatitis B and C treatments)," Medical Journal sya. Co., Ltd., 2008, pp. 19-25
[NPL 12] "Lancet", (the United Kingdom), 1998, Vol. 352, No. 9138, p. 1426-1432
[NPL 13] "Uirusu Kanen No Atarashii Chiryohou (New treatment method for viral hepatitis)", Medical View Co., Ltd., 2008, Vol. 25, No. 3, p. 76-81
[NPL 14] "Nature Reviews", (the United States), 2007, No. 6, p. 991-1000

### [Summary of Invention]

### [Technical Problem]

The present invention has been made in view of such circumstances, and an object of the present invention is to provide a novel triterpene derivative. In a preferred mode, an object of the present invention is to provide a triterpene derivative which exhibits a higher antiviral activity against chronic hepatitis C than conventional triterpene derivatives. In another preferred mode, an object of the present invention is to provide a triterpene derivative having favorable biokinetics. Moreover, an object of the present invention is to provide an agent for preventing or treating chronic hepatitis C, the agent comprising the triterpene derivative as an active ingredient.

### [Solution to Problem]

The present inventors have conducted earnest study to achieve the above objects. As a result, novel triterpene derivatives with a wide range of substituents were successfully synthesized. The synthesized triterpene derivatives were tested for anti-HCV action. As a result, it was found that multiple compounds exhibited a higher anti-HCV action than 22β-methoxyolean-12-ene-3β,24-diol, which has been conventionally used. Moreover, most of these compounds exhibited favorable biokinetics. Based on these characteristics, the present inventors have found that the identified triterpene derivatives can be excellent agents for preventing or treating chronic hepatitis C. This finding has led to the completion of the present invention.

Specifically, the present invention provides the following invention.
(1) A triterpene derivative represented by a general formula (I) or a pharmaceutically acceptable salt thereof: 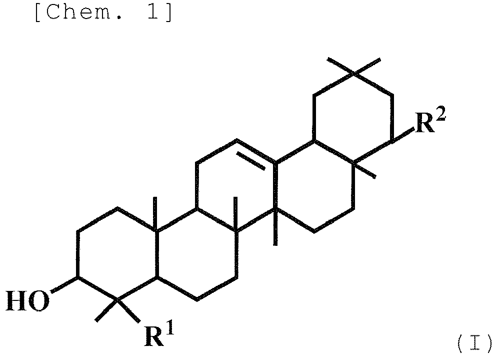 [in the formula (I), R¹ represents a carboxyl group, a hydroxymethyl group, -CH₂OSO₃H, or 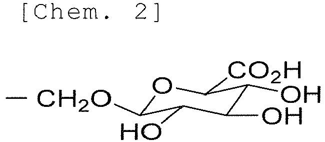 and R² represents -OR³ or -O-(CH₂)ₘ-OR⁴, where R³ represents a benzyl group which may be substituted with a hydroxymethyl group, a dimethylaminomethyl group, a phenylaminomethyl group, a morpholinomethyl group, a carboxyl group, or a formyl group, a C₁₋₆ alkyl group, a C₂₋₆ alkenyl group, a C₂₋₆ alkynyl group, or a hydroxy C₁₋₆ alkyl group, R⁴ represents a phenyl group which may be substituted with a carboxyl group, and m represents an integer of 1 to 3, with the proviso that a case where R¹ is a hydroxymethyl group, R² is -OR³, and R³ is a C₁₋₆ alkyl group, a C₂₋₆ alkenyl group, or a benzyl group is excluded].
(2) A triterpene derivative represented by a general formula (I) or apharmaceuticallyacceptable salt thereof: 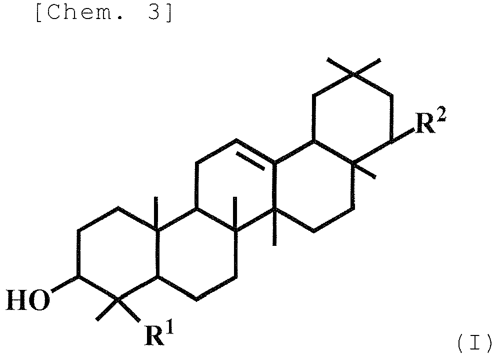 [in the formula (I), R¹ represents a carboxyl group, and R² represents -OR³, where R³ represents a benzyl group, a methyl group, or an allyl group].
(3) A triterpene derivative represented by a general formula (I) or a pharmaceutically acceptable salt thereof: 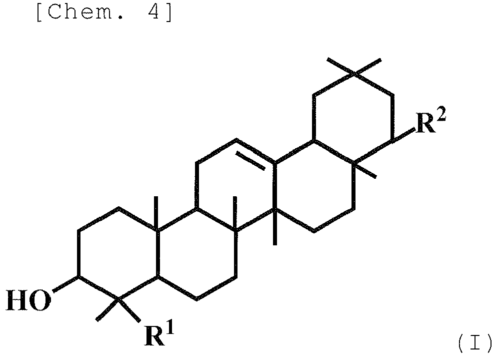 [in the formula (I), R¹ represents a hydroxymethyl group, and R² represents -OR³ or -O-(CH₂)₃-OR⁴, where R³ represents a benzyl group substituted with a hydroxymethyl group, a dimethylaminomethyl group, a phenylaminomethyl group, a morpholinomethyl group, a carboxyl group, or a formyl group, a 2-propynyl group, a 3-hydroxypropyl group, or a 2-hydroxypropyl group, and R⁴ represents a phenyl group or a phenyl group substituted with a carboxyl group].
(4) A triterpene derivative represented by a general formula (I) or a pharmaceutically acceptable salt thereof: 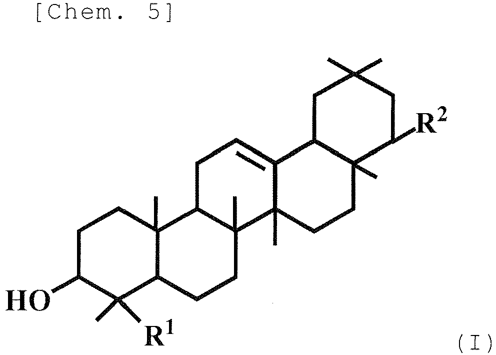 [in the formula, R¹ represents -CH₂OSO₃H, and R² represents a methoxy group].
(5) A triterpene derivative represented by a general formula (I) or apharmaceuticallyacceptable salt thereof: 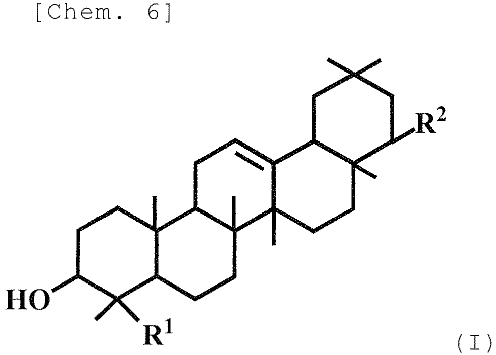 [in the formula (I), R¹ represents 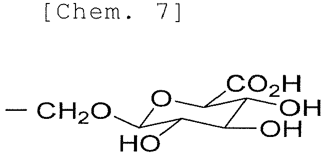 , and R² represents a methoxy group].
(6) The triterpene derivative according to any one of (1) to (5) or a pharmaceutically acceptable salt thereof, wherein an IC50 value of inhibitory activity against hepatitis C virus is 20 µM or less.
(7) An agent for preventing or treating chronic hepatitis C, comprising the triterpene derivative according to any one of (1) to (6) or a pharmaceutically acceptable salt thereof as an active ingredient.
(8) The agent according to (7), comprising a pharmaceutically acceptable carrier.
(9) The agent according to (7) or (8), further comprising an interferon in combination.
(10) A method for preventing or treating chronic hepatitis C, comprising administering the agent according to any one of (7) to (9) in a prophylactically or therapeutically effective amount to a patient who needs prevention or treatment of chronic hepatitis C.
(11) Use of the triterpene derivative or the pharmaceutically acceptable salt according to any one of (1) to (6), for producing an agent for preventing or treating chronic hepatitis C.
(12) The use according to (11), wherein the agent for preventing or treating chronic hepatitis C is the agent according to any one of (7) to (9).
(13) The triterpene derivative or the pharmaceutically acceptable salt according to any one of (1) to (6), for use in prevention or treatment of chronic hepatitis C.

### [Advantageous Effects of Invention]

The preventing or treating agent of the present invention comprises the triterpene derivative represented by the general formula (I) or a pharmaceutically acceptable salt thereof as an active ingredient. The triterpene derivative can exhibit an excellent anti-HCV activity or excellent biokinetics, when administered in an effective amount. Hence, a high prophylactic effect or a high therapeutic effect against chronic hepatitis C can be expected. In addition, a better prophylactic effect or a better therapeutic effect can also be expected, when the preventing or treating agent of the present invention is combined with any of IFN and ribavirin, which have been used clinically, or any of various anti-HCV drugs which will be used clinically in the future.

### [Description of Embodiments]

In the present invention, "chronic hepatitis C" means an inflammatory disease in the liver caused by persistent infection of a hepatitis C virus in the liver. In such Hepatitis C, the infection persists for 6 months or longer.

The triterpene derivative of the present invention can exhibit an excellent anti-HCV activity. The anti-HCV activity herein mainly refers to an activity to inhibit HCV proliferation. The lower the concentration (IC50 value) at which a 50% inhibition of HCV proliferation is achieved is, the higher anti-HCV activity is. A compound with an IC50 value exceeding 20 µM cannot be considered to have a high anti-HCV activity, and presumably has a low anti-virus effect, when administered alone for prevention or treatment of chronic hepatitis C. Accordingly, the triterpene derivative of the present invention is preferably one having a high anti-HCV activity, i.e., one having an IC50 value of 20 µM or less (for example, 15 µM or less, 10 µM or less, 8 µM or less, or 6 µM or less). Of the triterpene derivatives of the present invention, those having a particularly high anti-HCV activity (with an IC50 value of 10 µM or less) are Compounds 2 to 10 and Compounds 12 to 14, among Compounds listed in Table 1 shown later.

Moreover, the triterpene derivative of the present invention is preferably one having favorable biokinetics, i.e., one having an AUC of 50 µg·hr/ml or more (for example, 60 µg·hr/ml or more, 70 µg·hr/ml or more, or 80 µg·hr/ml or more), or one which has an AUC of 30 to 50 µg·hr/ml and which has a half life (t_{1/2}) of 10 hours or longer (for example, 11 hours or longer, 12 hours or longer, or 13 hours or longer). Of the triterpene derivatives of the present invention, compounds having favorable biokinetics are Compound 2 and 12 to 14.

Of the triterpene derivatives of the present invention, compounds having a particularly high anti-HCV activity and favorable biokinetics are Compounds 2 and 12 to 14.

An active ingredient in the preventing or treating agent of the present invention is a triterpene derivative represented by a general formula (I), or a pharmaceutically acceptable salt thereof.

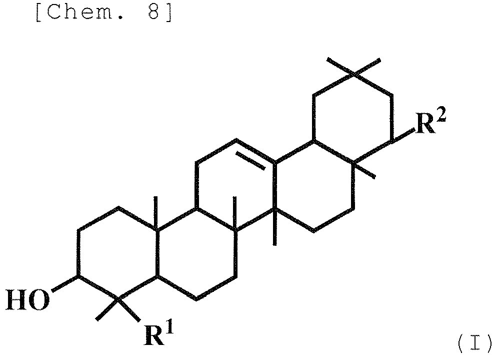

In an preferred mode, the triterpene derivative of the present invention is a compound represented by the formula (I), where R¹ is a carboxyl group, and R² represents -OR³, where R³ represents a benzyl group which may be substituted with a hydroxymethyl group, a dimethylaminomethyl group, a phenylaminomethyl group, a morpholinomethyl group, a carboxyl group, or a formyl group, a C₁₋₆ alkyl group, a C₂₋₆ alkenyl group, a C₂₋₆ alkynyl group, or a hydroxy C₁₋₆ alkyl group. Here, when substituted with any of the groups, "the benzyl group which may be substituted with a hydroxymethyl group, a dimethylaminomethyl group, a phenylaminomethyl group, a morpholinomethyl group, a carboxyl group, or a formyl group" represented by R³ may be substituted at any position, and is preferably a benzyl group substituted at position 4 with a hydroxymethyl group, a dimethylaminomethyl group, a phenylaminomethyl group, a morpholinomethyl group, a carboxyl group, or a formyl group. In addition, the "C₁₋₆ alkyl group" represented by R³ means a linear or branched alkyl group having 1 to 6 carbon atoms, and preferably 1 to 4 carbon atoms. Specific examples thereof include a methyl group, an ethyl group, a propyl group, an isopropyl group, a n-propyl group, a n-butyl group, an isobutyl group, a t-butyl group, and the like. Of these groups, preferred are a methyl group and an ethyl group, and more preferred is a methyl group. Meanwhile, the "C₂₋₆ alkenyl group" represented by R³ means a linear or branched alkenyl group having 2 to 6 carbon atoms, and preferably 2 to 4 carbon atoms. Specific examples thereof include a vinyl group, an allyl group, a 1-propenyl group, an isopropenyl group, a 1-butenyl group, a 2-butenyl group, a 3-butenyl group, a 2-methyl-1-propenyl group, and the like. Of these groups, preferred is an allyl group. In addition, the "C₂₋₆ alkynyl group" represented by R³ means a linear or branched alkynyl group having 2 to 6 carbon atoms, and preferably 2 to 4 carbon atoms. Specific examples thereof include a 2-propynyl group, a 1-methyl-2-propynyl group, a 2-butynyl group, a 3-butynyl group, and the like. Of these groups, preferred is a 2-propynyl group. Meanwhile, the "hydroxy C₁₋₆ alkyl group" represented by R³ may be substituted at any position with the hydroxyl group. Specific examples thereof include a hydroxymethyl group, a hydroxyethyl group, a 2-hydroxypropyl group, a 3-hydroxypropyl group, and the like. Of these groups, preferred is a 2-hydroxypropyl group or a 3-hydroxypropyl group.

In another preferred mode, the triterpene derivative of the present invention is a compound represented by the formula (I), where R¹ is a carboxyl group, and R² represents -O-(CH₂)m-OR⁴ (m represents an integer of 1 to 3), where R⁴ represents a phenyl group which may be substituted with a carboxyl group. Here, when substituted, the "phenyl group which may be substituted with a carboxyl group" represented by R⁴ in -O-(CH₂)m-OR⁴ represented by R² may be substituted at any position. The "phenyl group which may be substituted with a carboxyl group" is preferably a phenyl group or a phenyl group substituted at position 4 with a carboxyl group. In addition, a preferred integer of m is 3.

In still another preferred mode, the triterpene derivative of the present invention is a compound represented by the formula (I), where R¹ is a hydroxymethyl group, and R² represents -OR³, where R³ represents a benzyl group which may be substituted with a hydroxymethyl group, a dimethylaminomethyl group, a phenylaminomethyl group, a morpholinomethyl group, a carboxyl group, or a formyl group, a C₂₋₆ alkynyl group, or a hydroxy C₁₋₆ alkyl group. Here, the "benzyl group which may be substituted with a hydroxymethyl group, a dimethylaminomethyl group, a phenylaminomethyl group, a morpholinomethyl group, a carboxyl group, or a formyl group" represented by R³ may be substituted at any position, and is preferably a benzyl group substituted at position 4 with a hydroxymethyl group, a dimethylaminomethyl group, a phenylaminomethyl group, a morpholinomethyl group, a carboxyl group, or a formyl group. Meanwhile, the "C₂₋₆ alkynyl group" represented by R³ means a linear or branched alkynyl group having 2 to 6 carbon atoms, and preferably 2 to 4 carbon atoms. Specific examples thereof include a 2-propynyl group, a 1-methyl-2-propynyl group, a 2-butynyl group, a 3-butynyl group, and the like. Of these groups, preferred is a 2-propynyl group. In addition, the "hydroxy C₁₋₆ alkyl group" represented by R³ may be substituted at any position with the hydroxyl group. Specific examples thereof include a hydroxymethyl group, a hydroxyethyl group, a 2-hydroxypropyl group, a 3-hydroxypropyl group, and the like. Of these groups, preferred is a 2-hydroxypropyl group or a 3-hydroxypropyl group. A case where R¹ is a hydroxymethyl group, R² is -OR³, and R³ is a C₁₋₆ alkyl group, a C₂₋₆ alkenyl group, or a benzyl group is excluded from the triterpene derivative of the present invention.

In still another preferred mode, the triterpene derivative of the present invention is a compound represented by the formula (I), where R¹ is a hydroxymethyl group, and R² represents -O-(CH₂)m-OR⁴ (m represents an integer of 1 to 3), where R⁴ represents a phenyl group which may be substituted with a carboxyl group. Here, when substituted, the "phenyl group which may be substituted with a carboxyl group" represented by R⁴ in -O-(CH₂)mOR⁴ represented by R² may be substituted at any position. The "phenyl group which may be substituted with a carboxyl group" is preferably a phenyl group or a phenyl group substituted with at position 4 with a carboxyl group. In addition, a preferred integer of m is 3.

In still another preferred mode, the triterpene derivative of the present invention is a compound represented by the formula (I), where R¹ is -CH₂OSO₃H, and R² represents -OR³, where R³ represents a benzyl group which may be substituted with a hydroxymethyl group, a dimethylaminomethyl group, a phenylaminomethyl group, a morpholinomethyl group, a carboxyl group, or a formyl group, a C₁₋₆ alkyl group, a C₂₋₆ alkenyl group, a C₂₋₆ alkynyl group, or a hydroxy C₁₋₆ alkyl group. Here, when substituted with any of the groups, "the benzyl group which may be substituted with a hydroxymethyl group, a dimethylaminomethyl group, a phenylaminomethyl group, a morpholinomethyl group, a carboxyl group, or a formyl group" represented by R³ may be substituted at any position, and is preferably a benzyl group substituted at position 4 with a hydroxymethyl group, a dimethylaminomethyl group, a phenylaminomethyl group, a morpholinomethyl group, a carboxyl group, or a formyl group. Meanwhile, the "C₁₋₆ alkyl group" represented by R³ means a linear or branched alkyl group having 1 to 6 carbon atoms, and preferably 1 to 4 carbon atoms. Specific examples thereof include a methyl group, an ethyl group, a propyl group, an isopropyl group, a n-propyl group, a n-butyl group, an isobutyl group, a t-butyl group, and the like. Of these groups, preferred are a methyl group and an ethyl group, and more preferred is a methyl group. In addition, the "C₂₋₆ alkenyl group" represented by R³ means a linear or branched alkenyl group having 2 to 6 carbon atoms, and preferably 2 to 4 carbon atoms. Specific examples thereof include a vinyl group, an allyl group, a 1-propenyl group, an isopropenyl group, a 1-butenyl group, a 2-butenyl group, a 3-butenyl group, a 2-methyl-1-propenyl group, and the like. Of these groups, preferred is an allyl group. In addition, the "C₂₋₆ alkynyl group" represented by R³ means a linear or branched alkynyl group having 2 to 6 carbon atoms, and preferably 2 to 4 carbon atoms. Specific examples thereof include a 2-propynyl group, a 1-methyl-2-propynyl group, a 2-butynyl group, a 3-butynyl group, and the like. Of these groups, preferred is a 2-propynyl group. In addition, the "hydroxy C₁₋₆ alkyl group" represented by R³ may be substituted at any position with the hydroxyl group. Specific examples thereof include a hydroxymethyl group, a hydroxyethyl group, a 2-hydroxypropyl group, a 3-hydroxypropyl group, and the like. Of these groups, preferred is a 2-hydroxypropyl group or a 3-hydroxypropyl group.

In a still another preferred mode, the triterpene derivative of the present invention is a compound represented by the formula (I), where R¹ is -CH₂OSO₃H, and R² represents -O-(CH₂)m-OR⁴ (m represents an integer of 1 to 3), where R⁴ represents a phenyl group which may be substituted with a carboxyl group. Here, when substituted, the "phenyl group which may be substituted with a carboxyl group" represented by R⁴ in -O-(CH₂)m-OR⁴ represented by R² may be substituted at any position. The "phenyl group which may be substituted with a carboxyl group" is preferably a phenyl group or a phenyl group substituted at position 4 with a carboxyl group. In addition, a preferred integer of m is 3.

In still another preferred mode, the triterpene derivative of the present invention is a compound represented by the formula (I), where R¹ is 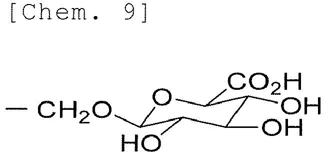 , and R² represents -OR³, where R³ is a benzyl group which may be substituted with a hydroxymethyl group, a dimethylaminomethyl group, a phenylaminomethyl group, a morpholinomethyl group, a carboxyl group, or a formyl group, a C₁₋₆ alkyl group, a C₂₋₆ alkenyl group, a C₂₋₆ alkynyl group, or a hydroxy C₁₋₆ alkyl group. Here, when substituted with any of the groups, "the benzyl group which may be substituted with a hydroxymethyl group, a dimethylaminomethyl group, a phenylaminomethyl group, a morpholinomethyl group, a carboxyl group, or a formyl group" represented by R³ may be substituted at any position, and is preferably a benzyl group substituted at position 4 with a hydroxymethyl group, a dimethylaminomethyl group, a phenylaminomethyl group, a morpholinomethyl group, a carboxyl group, or a formyl group. Meanwhile, the "C₁₋₆ alkyl group" represented by R³ means a linear or branched alkyl group having 1 to 6 carbon atoms, and preferably 1 to 4 carbon atoms. Specific examples thereof include a methyl group, an ethyl group, a propyl group, an isopropyl group, a n-propyl group, a n-butyl group, an isobutyl group, a t-butyl group, and the like. Of these groups, preferred are a methyl group and an ethyl group, and more preferred is a methyl group. In addition, the "C₂₋₆ alkenyl group" represented by R³ means a linear or branched alkenyl group having 2 to 6 carbon atoms, and preferably 2 to 4 carbon atoms. Specific examples thereof include a vinyl group, an allyl group, a 1-propenyl group, an isopropenyl group, a 1-butenyl group, a 2-butenyl group, a 3-butenyl group, a 2-methyl-1-propenyl group, and the like. Of these groups, preferred is an allyl group. In addition, the "C₂₋₆ alkynyl group" represented by R³ means a linear or branched alkynyl group having 2 to 6 carbon atoms, and preferably 2 to 4 carbon atoms. Specific examples thereof include a 2-propynyl group, a 1-methyl-2-propynyl group, a 2-butynyl group, a 3-butynyl group, and the like. Of these groups, preferred is a 2-propynyl group. In addition, the "hydroxy C₁₋₆ alkyl group" represented by R³ may be substituted at any position with the hydroxyl group. Specific examples thereof include a hydroxymethyl group, a hydroxyethyl group, a 2-hydroxypropyl group, a 3-hydroxypropyl group, and the like. Of these groups, preferred is a 2-hydroxypropyl group or a 3-hydroxypropyl group.

In a still another preferred mode, the triterpene derivative of the present invention is a compound represented by the formula (I), where R¹ is 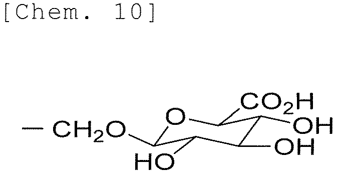 , and R² represents -O-(CH₂)m-OR⁴ (m represents an integer of 1 to 3), where R⁴ represents a phenyl group which may be substituted with a carboxyl group. Here, when substituted, the "phenyl group which may be substituted with a carboxyl group" represented by R⁴ in -O-(CH₂)m-OR⁴ represented by R² may be substituted at any position. The "phenyl group which may be substituted with a carboxyl group" is preferably a phenyl group or a phenyl group substituted at position 4 with a carboxyl group. In addition, a preferred integer of m is 3.

Each compound of the formula (I) has various isomers. The present invention encompasses any of these isomers and mixtures thereof. In addition, isomers due to other groups in the formula (I) may be present. The present invention also encompasses these isomers and mixtures thereof.

The triterpene derivative of the present invention preferably has a configuration represented by the formula (I-1). The definitions of R¹ and R² in the formula (I-1) are the same as those described above.

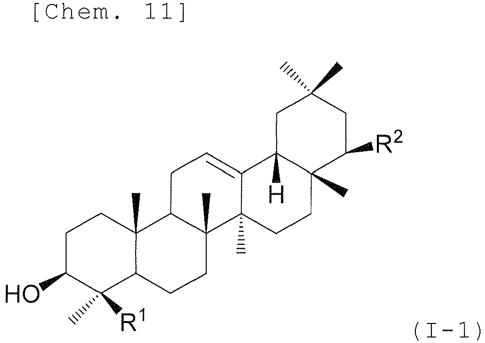

Moreover, in the present invention, specificexamples of the triterpene derivative of the formula (I) include compounds having combinations of substituents shown in Table 1. However, the present invention is not limited to these compounds.

**[Table 1]**

| Compound NO. | R¹ | R² |
|---|---|---|
| 1 | CH₂OH | 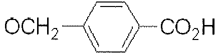 |
| 2 | CH₂OH | OCH₂C≡CH |
| 3 | CH₂OH | 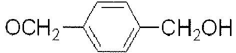 |
| 4 | CH₂OH | 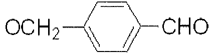 |
| 5 | CH₂OH | OCH₂CH(OH)CH₃ |
| 6 | CH₂OH | O(CH₂)₃OPh |
| 7 | CH₂OH | O(CH₂)₃OH |
| 8 | CH₂OH | 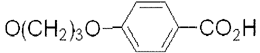 |
| 9 | CH₂OH | 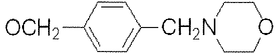 |
| 10 | CH₂OH | 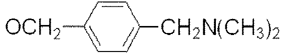 |
| 11 | CH₂OH | 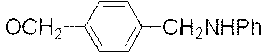 |
| 12 | CO₂H | OCH₂Ph |
| 13 | CO₂H | OCH₂CH=CH₂ |
| 14 | CO₂H | OCH₃ |
| 15 | CH₂OSO₃H | OCH₃ |
| 16 | 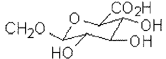 | OCH₃ |

Preferred methods for producing triterpene derivatives of the present invention represented by the general formula (I) are as follows. Note that, in the following production methods, functional groups which do not take part in the reaction are desirably protected, and known protective groups can be used for protecting the functional groups. These facts are evident to those skilled in the art.

### <Method (A) >

Of the triterpene derivatives represented by the general formula (I), compounds of the formula (Ia) can be produced by the following method.

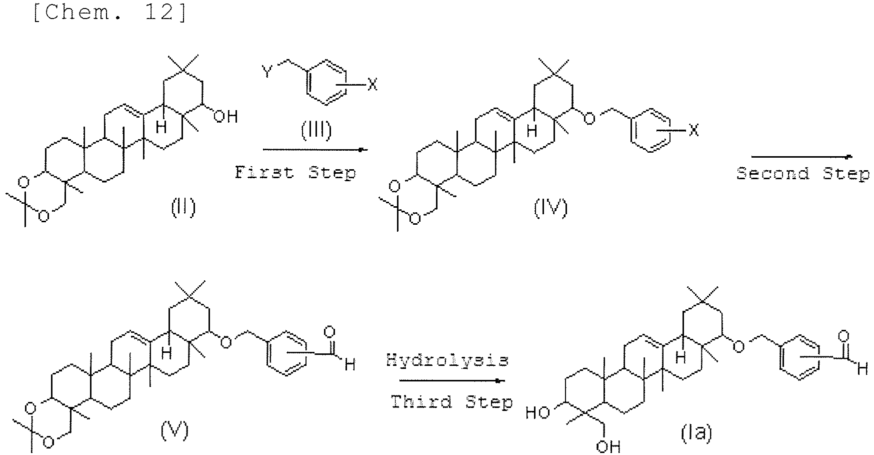

First, in First Step, a compound of the formula (II) (which can be synthesized by a method described in Chem. Pharm. Bull., 36. 153 (1988)) and a compound of the formula (III) (in the formula, X and Y are halogen atoms, which may be the same or different) are reacted with each other in the presence of a base to produce a compound of the formula (IV). The reaction is carried out in a solvent which does not take part in the reaction (for example, one or a mixture solvent of any of chloroform, dichloromethane, diethyl ether, THF, benzene, toluene, DMF, DMSO, and the like) at a temperature in a range from -78°C to 100°C. Examples of usable bases include pyridine, triethylamine, 4-dimethylaminopyridine, sodium hydride, potassium hydride, n-butyllithium, NaCH₂SOCH₃, tert-BuOK, tert-BuONa, and the like. The base and the compound of the formula (III) are each desirably used in a range from 1 to 10 equivalents to the compound of the formula (II).

Next, in Second Step, a halogen-metal exchange reaction is performed by treating the compound of the formula (IV) with an appropriate base, followed by a reaction with DMF. Thus, the compound (V) is produced. The reaction is carried out in a solvent which does not take part in the reaction (for example, one or a mixture solvent of any of tetrahydrofuran, diethyl ether, 1,4-dioxane, anisole, dimethoxyethane, dichloromethane, toluene, and the like) at a temperature in a range from -78°C to 30°C. Examples of usable bases include n-butyllithium, sec-butyllithium, tert-butyllithium, ethylmagnesium bromide, isopropylmagnesium bromide, and the like. The base and DMF are each desirably used in a range from 1 to 10 equivalents to the compound of the formula (IV).

Next, in Third Step, the compound represented by the formula (V) is subjected to a hydrolysis reaction in the presence of an acid. Thus, the compound represented by the formula (Ia) can be produced. Examples of solvents used for the reaction include one or a mixture solvent of any of methanol, ethanol, propanol, water, dichloromethane, chloroform, and THF; and the like. Examples of the acid include mineral acids such as hydrochloric acid and sulfuric acid; Lewis acids such as BF₃·OEt₂; and the like. The reaction is carried out at a temperature in a range from 0°C to 100°C.

### <Method (B)>

Of the triterpene derivatives represented by the general formula (I), compounds of the formula (Ib) can be produced by first reacting the compound of the formula (V) with an appropriate reducing agent in First Step, and subsequently performing a hydrolysis reaction in Second Step.

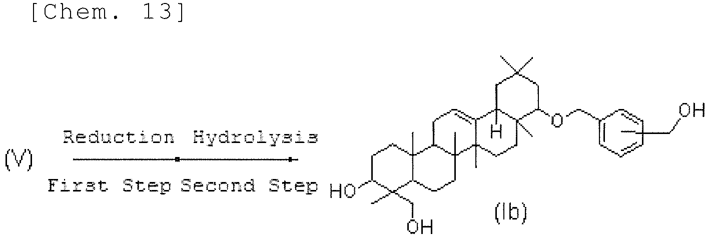

Examples of a solvent used in First Step include one or a mixture solvent of any of methanol, ethanol, THF, chloroform, dichloromethane, and the like; and the like. The reaction is carried out at a temperature in a range from -40°C to 30°C. In addition, examples of the reducing agent include lithium aluminum hydride, sodium borohydride, lithium borohydride, and the like. The reducing agent is desirably used in a range from 1 to 10 equivalents to the compound of the formula (V). Second Step can be conducted according to the method described in Third Step of the above-described Method (A).

### <Method (C)>

Of the triterpene derivatives represented by the general formula (I), compounds of the formula (Ic) (in the formula, R^{3c} is a benzyl substituted with a dimethylaminomethyl group, a phenylaminomethyl group, or a morpholinomethyl group) can be produced by first reacting the compound of the formula (V) according to an ordinary method of reductive aminoalkylation reaction in First Step, and subsequently performing a hydrolysis reaction in Second Step.

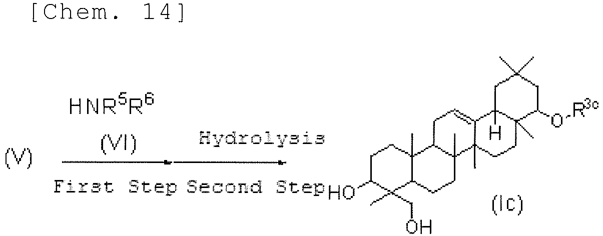

First, in First Step, the compound of the formula (V) and a compound of the formula (VI) (in the formula, HNR⁵R⁶ is dimethylamine, aniline, or morpholine) are reacted with each other according to an ordinary method of reductive aminoalkylation reaction. A Schiff base is obtained by a reaction using 1 equivalent, to the compound of the formula (V), of or an excessive amount of the compound of the formula (VI) in the presence of 1 equivalent or an excessive amount of an acid (for example, acetic acid, trifluoroacetic acid, or the like) in an inert solvent (for example, 1,2-dichloroethane, dichloromethane, or the like) at 0°C to 50°C. A reducing agent (for example, sodium borohydride, sodium cyanoborohydride, sodium triacetoxyborohydride, or the like) is added to the Schiff base, and a reaction is allowed to proceed at 0 °C to 50 °C. The reducing agent is desirably used in a range from 1 to 10 equivalents to the compound of the formula (V). Second Step can be conducted according to the method described in Third Step of the above-described Method (A).

### <Method (D) >

Of the triterpene derivatives represented by the general formula (I), compounds of the formula (Id) can be produced by first reacting the compound of the formula (V) according to an ordinary method of oxidation reaction in First Step, and subsequently performing a hydrolysis reaction in Second Step.

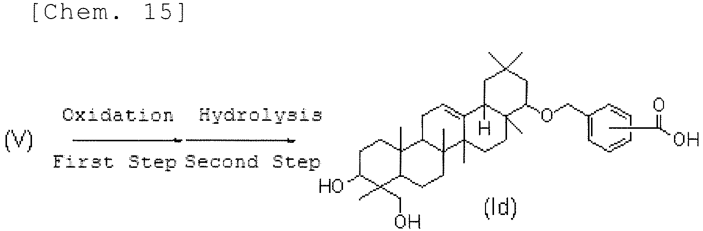

Examples of oxidizing agents usable for the oxidation reaction in First Step include pyridinium dichromate, Jones reagent, potassium permanganate, sodium chlorite, and the like. The oxidizing agent is preferably used in a range from 1 to 30 equivalents to the compound of the formula (V). The oxidation reaction is carried out in a solvent which does not take part in the reaction (for example, DMF, tert-butanol, acetone, water, or the like) at a temperature in a range from 0 °C to 60 °C. Second Step can be conducted according to the method described in Third Step of the above-described Method (A).

### <Method (E)>

Of the triterpene derivatives represented by the general formula (I), compounds of the formula (Ie) (in the formula, R^{3e} is a C₂₋₆ alkynyl group) can be produced by the following method. 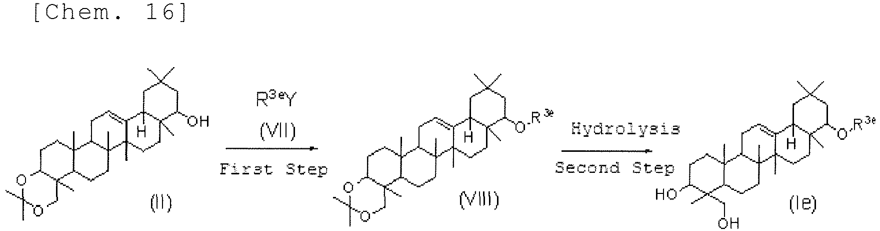 First, in First Step, the compound of the formula (II) and a compound of the formula (VII): R^{3e}Y (in the formula, R^{3e} has the same meaning as that described above, and Y is a halogen atom) are reacted with each other in the presence of a base. Thus, a compound of the formula (VIII) is produced. The reaction is carried out in a solvent which does not take part in the reaction (for example, one or a mixture solvent of any of chloroform, dichloromethane, diethyl ether, THF, benzene, toluene, DMF, DMSO, and the like) at a temperature in a range from -78 °C to 100 °C. Examples of usable bases include pyridine, triethylamine, 4-dimethylaminopyridine, sodium hydride, potassium hydride, n-butyllithium, NaCH₂SOCH₃, tert-BuOK, tert-BuONa, and the like. The base and the compound of the formula (VII) are each desirably used in a range from 1 to 10 equivalents to the compound of the formula (II). Second Step can be conducted according to the method described in Third Step of the above-described Method (A).

### <Method (F)>

Of the triterpene derivatives represented by the general formula (I), Compound 5 and Compound 7 can be produced by the following method.

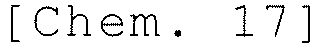

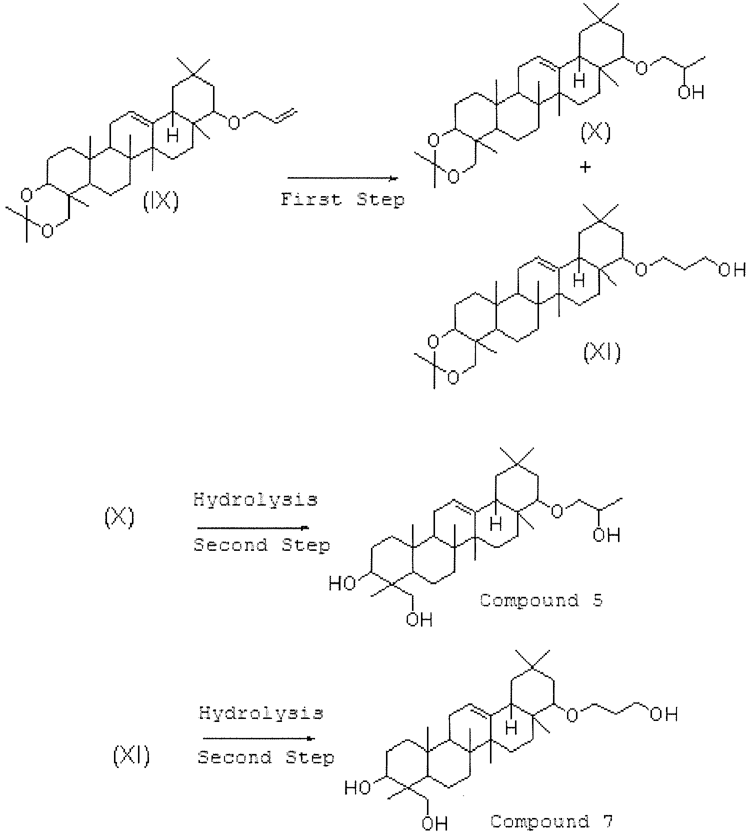

First, in First Step, a compound of the formula (IX) (which can be synthesized by a method described in DESCRIPTION of Japanese Patent No. 3279574) is reacted with a hydroboration reagent, followed by oxidation. Thus, compounds of the formula (X) and the formula (XI) are obtained. Examples of the hydroboration reagent include BH₃-THF, thexylborane, 9-borabicyclo(3,3,1) nonane, and the like. The reagent is preferably used in a range from 1 to 10 equivalents to the compound of the formula (IX). The reaction is carried out in a solvent which does not take part in the reaction (for example, diethyl ether, THF, or the like) at a temperature in a range from 0°C to 50°C. In addition, the oxidation reaction is carried out at a temperature in a range from 0°C to 40°C, with an oxidizing agent (for example, sodium hydroxide, 30% aqueous hydrogen peroxide) being added to the reaction liquid. Second Step can be conducted according to the method described in Third Step of the above-described Method (A).

### <Method (G)>

Of the triterpene derivatives represented by the general formula (I), compounds of the formula (Ig) (in the formula, R⁴ and m have the same meanings as described above) can be produced by the following method.

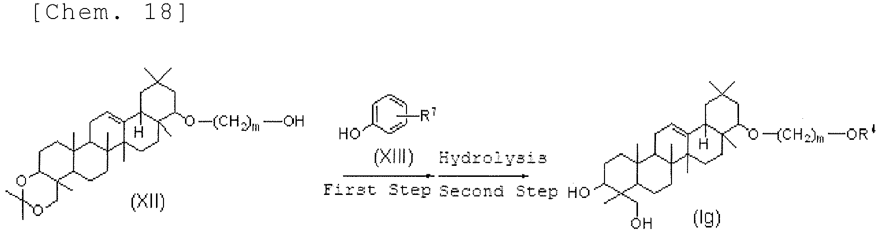

First, in First Step, a compound of the formula (XII) (which can be synthesized by the above-described Method F or the like) and a compound of the formula (XIII) (in the formula, R⁷ is a hydrogen atom or a carboxylic acid methyl ester) are reacted with each other according to an ordinary method of the Mitsunobu reaction. Specifically, the reaction is conducted by using 1 equivalent, to the compound of the formula (XII), of or an excessive amount of the compound of the formula (XIII) in the presence of 1 equivalent or an excessive amount of a phosphine reagent (for example, triphenylphosphine) and 1 equivalent or an excessive amount of an azo reagent (for example, diethyl azodicarboxylate, 1,1'-(azodicarbonyl)dipiperidine, or the like) in a solvent which does not take part in the reaction (for example, THF, dichloromethane, toluene, benzene, or the like) at 0°C to 50°C.

When R⁷ is a hydrogen atom, Second Step can be conducted according to the method described in Third Step of the above-described Method (A). Meanwhile, when R⁷ is a carboxylic acid methyl ester, a hydrolysis reaction of the methyl ester moiety is first conducted by using a base, and then a hydrolysis reaction is conducted in the presence of an acid, in Second Step. The hydrolysis reaction of the methyl ester using the base is carried out by reacting the methyl ester with 1 equivalent of to an excessive amount of the base (for example, sodium hydroxide, potassium hydroxide, lithium hydroxide, barium hydroxide, tert-BuOK, or the like) in a polar solvent (for example, one or a mixture solvent of any of methanol, ethanol, propanol, water, and the like) at a temperature in a range from 0°C to 100°C. The hydrolysis reaction subsequently conducted in the presence of the acid is conducted by adding the acid (a mineral acid such as hydrochloric acid or sulfuric acid, a Lewis acid such as BF₃·OEt₂, or the like), in a one-pot manner, to a reaction system in which the hydrolysis of the methyl ester is completed, until the reaction system becomes acidic. In addition, the reaction here is carried out at a temperature in a range from 0°C to 100°C.

### <Method (H) >

Of the triterpene derivatives represented by the general formula (I), compounds of the formula (Ih) (in the formula, R^{3h} is a C₁₋₆ alkyl group, a C₂₋₆ alkenyl group, a C₂₋₆ alkynyl group, or benzyl) can be produced by the following method.

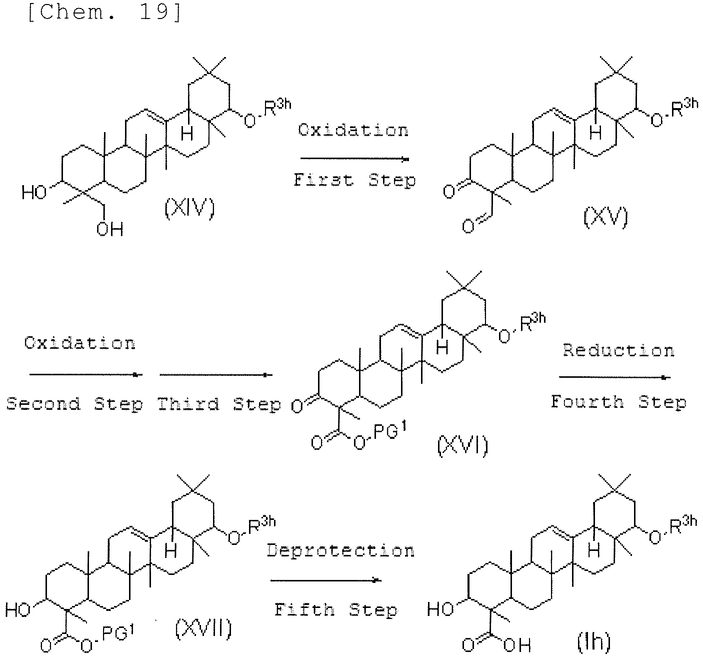

First, in First Step, a compound of the formula (XIV) (which can be synthesized by a method described in DESCRIPTION of Japanese Patent No. 3279574, DESCRIPTION of Japanese Patent No. 3727353, or Bioorganic & Medicinal Chemistry, 13, 4900 (2005), or the like) is converted to a compound of the formula (XV) by oxidation. Examples of usable oxidizing agents include (1) pyridiniumchloromate, (2) pyridinium dichromate, (3) manganese dioxide, (4) a combination of tetra-n-propylammonium perruthenate and N-methylmorpholine-N-oxide, (5) a DMSO oxidation reagent such as a combination of dimethyl sulfoxide (DMSO) and oxalyl chloride, and the like. The oxidizing agent is preferably used in a range from 2 to 10 equivalents to the compound of the formula (XIV). The oxidation reaction can be carried out in a solvent which does not take part in the reaction (for example, dichloromethane, chloroform, diethyl ether, THF, or the like) at a temperature in a range from -78°C to 40°C.

Subsequently, in Second Step, the compound of the formula (XV) is oxidized, and then the formed carboxylic acid is protected with an ordinary protective group in Third Step, and is thus converted into a compound of the formula (XVI) (where PG¹ represents an ordinary protective group for a carboxyl group; here, the ordinary protective group for a the carboxyl group represents any of those described in Protective Groups in Organic Synthesis (written by Theodora W. Greene, Peter G. M. Wuts, published by John Wiley & Sons, Inc.), and those protective groups are well-known to those skilled in the art; and further preferably, PG¹ is a methyl, ethyl, or benzyl group, or the like). Examples of oxidizing agents usable for the oxidation reaction in Second Step include pyridinium dichromate, Jones reagent, potassium permanganate, sodium chlorite, and the like. The oxidizing agent is preferably used in a range from 1 to 30 equivalents to the compound of the formula (XV). The oxidation reaction is carried out in a solvent which does not take part in the reaction (for example, one or a mixture solvent of any of DMF, tert-butanol, acetone, water, and the like) at a temperature in a range from 0°C to 60°C. Conditions for the protection of the carboxylic acid in Third Step vary depending on the kind of the protective group used. For example, when PG¹ is methyl, ethyl, or benzyl, the protection can be carried out by a reaction using 1 equivalent to 10 equivalents, to the compound of the formula (XV), of PG¹Y (where Y is a halogen atom) in the presence of 1 equivalent of to an excessive amount of a base (for example, potassium carbonate, sodium carbonate, cesium carbonate, sodium hydride, or the like) in a solvent which does not take part in the reaction (for example, toluene, benzene, THF, DMF, DMSO, or the like) at a temperature in a range from room temperature to 100°C. Meanwhile, when PG¹ is a methyl, the protection can be carried out by a reaction using 1 equivalent to 10 equivalents, to the compound of the formula (XV), of trimethylsilyldiazomethane in a mixture solvent of a non-polar solvent and an alcohol solvent (for example, toluene with methanol, benzene with methanol, or the like) at a temperature in a range from 0°C to 50°C.

Subsequently, in Fourth Step, the formula (XVI) is reduced by an ordinary reduction reaction, and is thus converted to a compound of the formula (XVII). Examples of solvents used in the reduction reaction include one or a mixture solvent of any of methanol, ethanol, THF, chloroform, dichloromethane, and the like; and the like. As for a reaction temperature, the reaction is carried out at a temperature in a range from -78°C to 30 °C. In addition, examples of the reducing agent include lithium aluminum hydride, sodium borohydride, lithium borohydride, diisobutylaluminum hydride, and the like. The reducing agent is desirably used in a range from 1 to 10 equivalents to the compound of the formula (XVI).

Subsequently, in Fifth Step, PG¹ of the compound of the formula (XVII) is removed under ordinary deprotection conditions of the protective group for a carboxyl group. Thus, a compound of the formula (Ih) is produced. At this time, the deprotection conditions for removing the protective group vary depending on the kind of the protective group used. For example, when PG¹ is methyl, ethyl, or benzyl, the deprotection is carried out by a reaction using 1 equivalent of to an excessive amount of abase (forexample, sodium hydroxide, potassium hydroxide, lithium hydroxide, barium hydroxide, tert-BuOK, or the like) in a polar solvent (for example, one or a mixture solvent of any of methanol, ethanol, propanol, water, and the like) at a temperature in a range from 0°C to 100°C. Meanwhile, when PG¹ is benzyl, the deprotection can be carried out by catalytic reduction using 0.1 to 0.5 equivalents of a catalyst (for example, palladium carbon, palladium black, palladium hydroxide, or the like). The reaction can be carried out in a solvent which does not take part in the reaction (for example, methanol, ethanol, THF, dioxane, dichloromethane, chloroform, or water), generally, under a hydrogen atmosphere of 1 to 4 atm at room temperature.

### <Method (I)>

Of the triterpene derivatives represented by the general formula (I), the compounds of the formula (Ii) (in the formula, R³ⁱ is a C₁₋₆ alkyl group, a C₂₋₆ alkenyl group, or a C₂₋₆ alkynyl group) can also be produced by the following method.

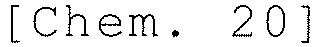

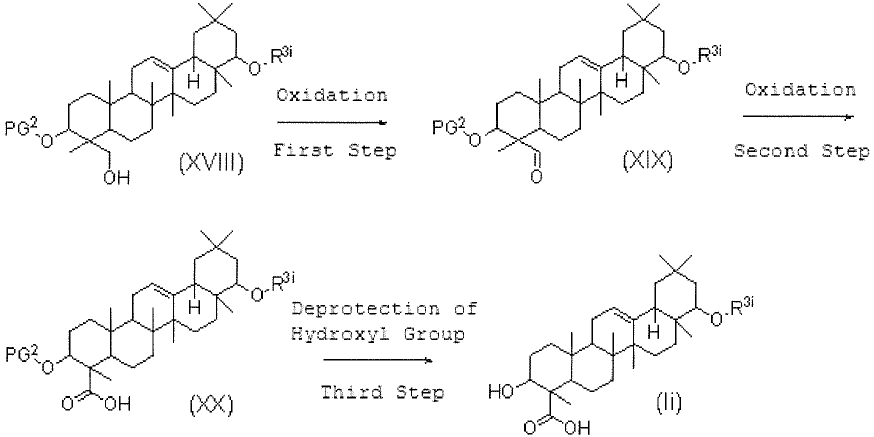

First, in First Step, a compound of the formula (XVIII) is oxidized by the method described in First Step of Method (H), and is thus converted to a compound of the formula (XIX). In the compound of the formula (XVIII), PG² represents an ordinary protective group for a hydroxyl group. The "ordinary protective group for a hydroxyl group" represents any of those described in Protective Groups in Organic Synthesis (written by Theodora W. Greene, Peter G. M. Wuts, published by John Wiley & Sons, Inc.), and these protective groups are well-known to those skilled in the art. In the reaction, PG² is preferably benzyl. The compound of the formula (XVIII) can be synthesized by a method described in DESCRIPTION of Japanese Patent No. 3279574, DESCRIPTION of Japanese Patent No. 3727353, or Bioorganic & Medicinal Chemistry, 13, 4900 (2005), or the like.

Subsequently, in Second Step, conversion to a compound of the formula (XX) is conducted by oxidation based on the method described in Second Step of Method (H).

Subsequently, in Third Step, the protective group is removed under ordinary deprotection conditions of the protective group for a hydroxyl group. Thus, a compound of the formula (Ii) is produced. The deprotection conditions for removing the protective group for a hydroxyl group in Third Step vary depending on the kind of the protective group used. For example, when PG² is benzyl, the deprotection can be carried out by catalytic reduction using 0.1 to 0.5 equivalents of a catalyst (for example, palladium carbon, palladium black, palladium hydroxide, or the like). The reaction can be carried out in a solvent which does not take part in the reaction (for example, methanol, ethanol, THF, dioxane, dichloromethane, chloroform, or water), generally, under a hydrogen atmosphere of 1 to 4 atm at room temperature.

### <Method (J) >

Of the triterpene derivatives represented by the general formula (I), the compounds of the formula (Ij) (in the formula, R³ⁱ has the same meaning as that described above) can also be produced by the following method.

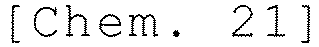

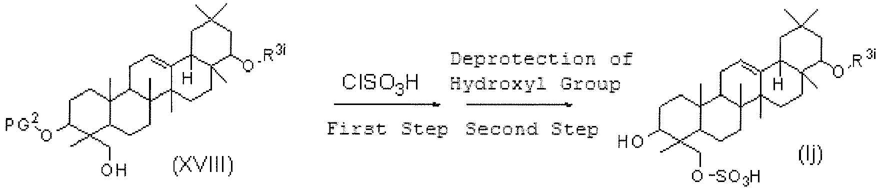

First, in First Step, the compound of the formula (XVIII) (in the formula, PG² has the same meaning as that described above, and is preferably benzyl in this reaction) and 1 equivalent or an excessive amount of chlorosulfonic acid are reacted with each other in a solvent which does not take part in the reaction (for example, THF, dioxane, dichloromethane, toluene, benzene, or the like) at a temperature in a range from -20°C to 50°C.

Subsequently, in Second Step, the protective group is removed under ordinary deprotection conditions of a protective group for a hydroxyl group. Thus, a compound of the formula (Ij) is produced. The deprotection conditions for removing the protective group for a hydroxyl group in Second Step vary depending on the kind of the protective group used. For example, when PG² is benzyl, the protection can be carried out by catalytic reduction using 0.1 to 0.5 equivalents of a catalyst (for example, palladium carbon, palladium black, palladium hydroxide, or the like). The reaction can be carried out in a solvent which does not take part in the reaction (for example, one or a mixture solvent of any of methanol, ethanol, THF, dioxane, dichloromethane, chloroform, and water), generally, under a hydrogen atmosphere of 1 to 4 atm at room temperature.

### <Method (K) >

Of the triterpene derivatives represented by the general formula (I), compounds of the formula (Ik) (in the formula, R³ⁱ has the same meaning as that described above) can also be produced by the following method.

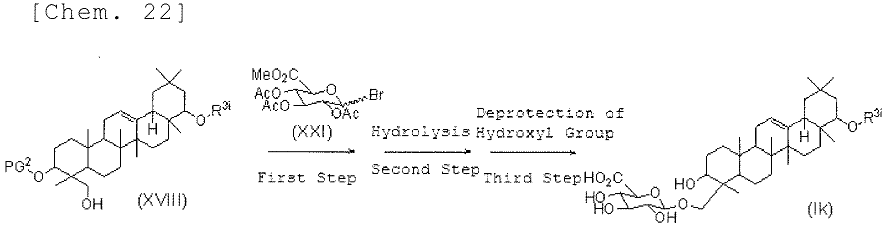

First, in First Step, a compound of the formula (XVIII) (in the formula, PG² has the same meaning as that described above, and is preferably benzyl in this reaction) is reacted with a compound of the formula (XXI) under ordinary conditions for glycosylation reaction. This first step is carried out by a reaction using 1 equivalent, to the compound of the formula (XVIII), of or an excessive amount of the compound of the formula (XXI) in the presence of 1 equivalent or an excessive amount of a bromosugar activator (for example, silver triflate, silver carbonate, or the like) in an inert solvent (for example, 1,2-dichloroethane, dichloromethane, benzene, toluene, or the like) at a temperature in a range from 0°C to 100°C.

Subsequently, in Second Step, a hydrolysis reaction of the acetyl groups and the methyl ester moiety is conducted by using a base. This Second Step is carried out by a reaction in the presence of 1 equivalent of to an excessive amount of the base (for example, sodium hydroxide, potassium hydroxide, lithium hydroxide, barium hydroxide, tert-BuOK, or the like) in a polar solvent (for example, one or a mixture solvent of any of methanol, ethanol, propanol, water, and the like) at a temperature in a range from 0°C to 100°C.

Subsequently, in Third Step, the protective group is removed under ordinary deprotection conditions of the protective group for a hydroxyl group, and thus a compound of the formula (Ik) is produced. The deprotection conditions for removing the protective group for a hydroxyl group in Third Step vary depending on the kind of the protective group used. For example, when PG² is benzyl, the deprotection can be carried out by catalytic reduction using 0.1 to 0.5 equivalents of a catalyst (for example, palladium carbon, palladium black, palladium hydroxide, or the like). The reaction can be carried out in a solvent which does not take part in the reaction (for example, methanol, ethanol, THF, dioxane, dichloromethane, chloroform, or water), generally, under a hydrogen atmosphere of 1 to 4 atm at room temperature.

The triterpene derivative represented by the above-described (I) of the present invention can be present as a salt. The salt can be easily obtained by reacting a pharmaceutically acceptable base with the triterpene derivative of the present invention according to an ordinary method. For example, an inorganic base such as sodium hydroxide, potassium hydroxide, aluminum hydroxide, sodium carbonate, potassium carbonate, or sodium hydrogen carbonate, or an organic base such as piperazine, morpholine, piperidine, ethylamine, or trimethylamine can be used as the base.

The triterpene derivative of the present invention can be used as an agent for preventing or treating chronic hepatitis C. The preventing or treating agent of the present invention can be administered orally, for example, in a dosage form such as a capsule, a microcapsule, a tablet, a granule, a fine granule, a powder, or the like. Further, the preventing or treating agent can also be parenterally administered (for example, intravenous injection, intramuscular injection, subcutaneous administration, intraperitoneal administration, rectal administration, and percutaneous administration) in a form of a conventional pharmaceutical preparation. Pharmaceutically acceptable carriers can be added to the above-described dosage forms. Examples of the pharmaceutically acceptable carriers include pharmaceutical additives such as excipients, fillers, binders, wetting agents, disintegrating agents, surfactants, lubricants, dispersants, buffers, preservatives, solubilizers, antiseptics, flavor modifiers, soothing agents, and stabilizers. Each dosage form can be produced in a usual manner. Specific examples of the additives include lactose, fructose, glucose, starch, gelatin, magnesium carbonate, synthetic magnesium silicate, talc, magnesium stearate, microcrystalline cellulose, methyl cellulose, carboxymethyl cellulose or a salt thereof, gum arabic, polyethylene glycol, syrup, vaseline, glycerin, ethanol, propylene glycol, citric acid, sodium chloride, sodium sulfite, sodium phosphate, and the like.

The dosage form, the administration method, the administration amount, the administration period, the administration route, and the like of the preventing or treating agent of the present invention can be set as appropriate in accordance with, for example, the body weight, the age, the severity of symptoms of the patient, the viral load in the patient, and the like. The preventing or treating agent of the present invention is administered orally or parenterally, for example, at 1 to 1000 mg/day as a single dose or plural divided doses. The administration amount is preferably 5 to 500 mg/day.

The preventing or treating agent of the present invention can be used in combination with another agent having an anti-viral effect, such as IFN or ribavirin, in some cases. The combination of the preventing or treating agent of the present invention with another agent may be in the form of individual pharmaceutical preparations, or may be a single agent comprising the active ingredients of both of the agents. In the case of the form of the individual pharmaceutical preparations, the agents can be administered simultaneously or with an interval, and the times of administration of the agents may be the same or different from each other.

Examples of the "interferon" combined in the preventing or treating agent of the present invention include IFN derivatives used for treatment, such as natural IFN-α (Sumiferon: manufactured by Dainippon Sumitomo Pharma Co., Ltd., etc.), IFN-α-2a, IFN-α-2b (Intron A: manufactured by Schering-Plough Corporation), polyethylene glycolated (PEGylated) natural IFN-α, PEGylated IFN-α-2a (Pegasys: manufactured by Roche Holding AG and Chugai Pharmaceutical Co., Ltd.), PEGylated IFN-α-2b (PEG-intron A: manufactured by Schering-Plough Corporation), natural IFN-β (IFN-β Mochida: manufactured by Mochida Pharmaceutical Co., Ltd., and Feron: manufactured by Toray Industries, Inc.), PEGylated IFN-β, natural IFN-γ, consensus IFN (Advaferon: manufactured by Astellas Pharma Inc., and the like), PEGylated consensus IFN, and long-lasting IFN. However, the "interferon" is not limited to these examples. Preferred is IFN-α-2b or IFN-α-2a. These IFNs are preferably PEGylated.

In general, IFN is administered subcutaneously, intravenously, or intramuscularly. However, other parenteral methods (for example, by a nasal spray, through the skin, by a suppository, or the like) are also possible. Moreover, oral administration is also possible, when an IFN effective in an oral method, such as an oral IFN, is used.

A ratio (a use ratio or a blending ratio) of the active ingredients in the combined agent is not particularly limited, as long as the ratio is effective for preventing or treating a chronic hepatitis C case. For example, the dose of the triterpene derivative of the present invention is generally determined as appropriate within a range from 1 mg to 1000 mg/day, with respect to an effective dose of the IFN. The dose of the triterpene derivative of the present invention is preferably in a range from 5 mg/day to 500 mg/day. In the case of a combination of the preventing or treating agent of the present invention with another agent, an appropriate administration amounts and administration intervals can be determined by a controlled clinical study.

### [Examples]

Hereinafter, the present invention will be described specifically based on Examples. However, these Examples do not limit the scope of the present invention.

Compounds described in Table 2 shown below were synthesized. Note that Compound 17 for comparison was synthesized by a method described in DESCRIPTION of Japanese Patent No. 3279574.

**[Table 2]**

| Compound NO. | R^{*1} | R^{*2} |
|---|---|---|
| 1 | CH₂OH | 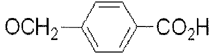 |
| 2 | CH₂OH | OCH₂C≡CH |
| 3 | CH₂OH | 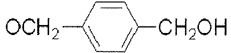 |
| 4 | CH₂OH | 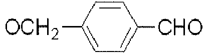 |
| 5 | CH₂OH | OCH₂CH(OH)CH₃ |
| 6 | CH₂OH | O(CH₂)₃OPh |
| 7 | CH₂OH | O(CH₂)₃OH |
| 8 | CH₂OH | 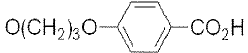 |
| 9 | CH₂OH | 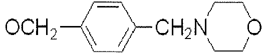 |
| 10 | CH₂OH | 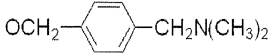 |
| 11 | CH₂OH | 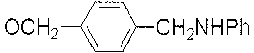 |
| 12 | CO₂H | OCH₂Ph |
| 13 | CO₂H | OCH₂CH=CH₂ |
| 14 | CO₂H | OCH₃ |
| 15 | CH₂OSO₃H | OCH₃ |
| 16 | 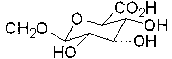 | OCH₃ |
| 17 | CH₂OH | OMe |

### [Example 1]

### Method for producing 22β-(4-carboxybenzyloxy)olean-12-ene-3β,24-diol (Compound 1)

### (Step 1)

### 22β-(4-iodobenzyloxy)-3β,24-isopropylidenedioxyolean-1 2-ene

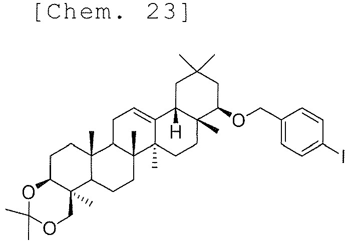

In 23 ml of DMF, 1.574 g of 22β-hydroxy-3β,24-isopropylidenedioxyolean-12-ene (which was synthesized by a method described in Chem. Pharm. Bull., 36. 153 (1988)) was dissolved, and 636 mg of 60% sodium hydride was added thereto, followed by stirring at room temperature for 15 minutes. To this mixture, 1.167 g of tetra-N-butylammonium iodide and 1.891 g of 4-iodobenzyl bromide were added, and the resultant mixture was heated to 70°C and stirred for 1 hour. Further, 1.891 g of 4-iodobenzyl bromide was added thereto, followed by stirring for 10 minutes. Then, the mixture was cooled to room temperature, and the reaction was quenched with a saturated aqueous solution of ammonium chloride. The reaction solution was diluted with ethyl acetate, then washed three times with saturated aqueous sodium chloride, and dried over anhydrous magnesium sulfate. The inorganic salt was separated by filtration, and the solvent was concentrated in a vacuum. The obtained residue was purified by silica gel column chromatography (development system, n-hexane:ethyl acetate=49:1). Thus, 1.535 g (yield: 68%) of the title compound of Step 1 was obtained. ¹H-NMR (CDCl₃) : 0.89 (3H, s), 0.92 (3H, s), 0.99 (3H, s), 1.03 (3H, s), 1.12 (3H, s), 1.15 (3H, s), 1.22 (3H, s), 1.38 (3H, s), 1.44 (3H, s), 0.87-2.18 (21H, m), 3.05 (1H, dd, J=2.9, 6.1 Hz), 3.23 (1H, d, J=11.6 Hz), 3.46 (1H, dd, J=4.6, 9.3 Hz), 4.05 (1H, d, J=11.6Hz), 4.25 (1H, d, J=12.0 Hz), 4.55 (1H, d, J=12.0 Hz), 5.24 (1H, t-like), 7.07-7.10 (2H, m), 7.62-7.66 (2H, m).
MS FAB (m/z): 715 (M⁺+1)

### (Step 2)

### 22β-(4-formylbenzyloxy)-3β,24-isopropylidenedioxyolean -12-ene

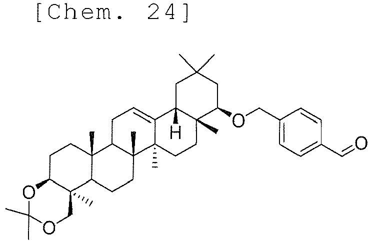

In 7.1 ml of THF, 506 mg of 22β-(4-iodobenzyloxy)-3β,24-isopropylidenedioxyolean-1 2-ene was dissolved, and the solution was cooled to -78°C. After addition of 0.31 ml of n-butyllithium (2.77 M, hexane solution) and stirring for 5 minutes, 0.27 ml of DMF was added. After stirring at -78°C for 20 minutes, the temperature was raised to -40°C over 15 minutes. The reaction was quenched with saturated aqueous ammonium chloride. The reaction solution was diluted with ethyl acetate, and then washed with water and saturated aqueous sodium chloride in this order. After drying over anhydrous magnesium sulfate, the inorganic salt was separated by filtration, and the solvent was concentrated in a vacuum. The obtained residue was purified by silica gel column chromatography (development system, n-hexane:ethyl acetate=49:1 to 19:1). Thus, 186 mg (42%) of the title compound of Step 2 was obtained.
¹H-NMR (CDCl₃): 0.91 (3H, s), 0.96 (3H, s), 1.00 (3H, s), 1.05 (3H, s), 1.13 (3H, s), 1.16 (3H, s), 1.22 (3H, s), 1.38 (3H, s), 1.44 (3H, s), 0.83-2.05 (20H, m), 2.19 (1H, m), 3. 10 (1H, dd, J=2.9, 6.1 Hz), 3.23 (1H, d, J=11.5 Hz), 3.45 (1H, dd, J=4.7, 9.5 Hz), 4.07 (1H, d, J=11.5 Hz), 4.40 (1H, d, J=12.9Hz), 4.70 (1H, d, J=12.9Hz), 5.26 (1H, t-like), 7.50 (2H, d, J=8. 1 Hz), 7.84 (2H, d, J=8. Hz), 10.0 (1H, s).
MS FAB (m/z): 617 (M⁺+1)

### (Step 3)

### 22β-(4-carboxybenzyloxy)olean-12-ene-3β,24-diol (Compound 1)

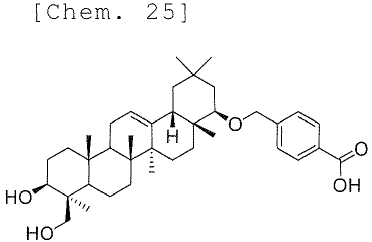

In a liquid mixture of 3 ml of tert-butanol and 1 ml of THF, 186 mg of 22β-(4-formylbenzyloxy)-3β,24-isopropylidenedioxyolean -12-ene was dissolved, and 0.32 ml of 2-methyl-2-butene was added thereto. To this solution, a solution of 143 mg of sodium dihydrogen phosphate dihydrate and 83 mg of sodium chlorite in 0.75 ml of water was added slowly, followed by stirring at room temperature for 1.5 hours. The reaction solution was diluted with chloroform, and then washed with a liquid mixture of a 1 N aqueous solution of hydrochloric acid and saturated aqueous sodium chloride. Extraction was conducted again from the aqueous layer with chloroform, and the combined organic layers were dried over anhydrous magnesium sulfate. Then, the inorganic salt was separated by filtration, and the solvent was concentrated in a vacuum. The obtained residue was dissolved in a liquid mixture of 5 ml of THF and 1 ml of water, and 0.3 ml of a 1 N aqueous solution of hydrochloric acid was added thereto. The solution was stirred at room temperature for 30 minutes, then diluted with saturated aqueous sodium chloride, and extracted three times with chloroform. The combined organic layers were dried over anhydrous magnesium sulfate. Then, the inorganic salt was separated by filtration, and the solvent was concentrated in a vacuum. The obtained residue was purified by silica gel column chromatography (development system, chloroform:methanol=49:1 to 97:3). Thus, 145 mg (yield: 81%) of Compound 1 was obtained.
¹H-NMR (CDCl₃) : 0.89 (3H, s), 0.90 (3H, s), 0.95 (6H, s), 1.04 (3H, s), 1.12 (3H, s), 1.25 (3H, s), 0.79-2.20 (21H, m), 3.09 (1H, m), 3.36 (1H, d, J=11.2 Hz), 3.45 (1H, dd, J=4.4, 11.7Hz), 4.22 (1H, d, J=11.2Hz), 4.38 (1H, d, J=12.8 Hz), 4.68 (1H, d, J=12.8 Hz), 5.24 (1H, t-like), 7.44 (2H, d, J=7.9 Hz), 8.06 (2H, d, J=7.9 Hz).
MS ES (m/z): 593 (M⁺+1)

### [Example 2] Method for producing 22β-(2-propynyloxy)olean-12-ene-3β,24-diol (Compound 2)

### (Step 1)

### 22β-(2-propynyloxy)-3β,24-isopropylidenedioxyolean-12-ene

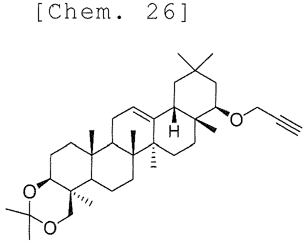

In 4 ml of DMF, 203 mg of 22β-hydroxy-3β,24-isopropylidenedioxyolean-12-ene was dissolved, and 162 mg of 60% sodium hydride was added thereto, followed by stirring at room temperature for 15 minutes. To this mixture, 151 mg of tetra-N-butylammonium iodide and 0.31 ml of propargyl bromide were added, followed by stirring at room temperature for 3 days. The reaction was quenched with water, and the reaction solution was diluted with ethyl acetate. Then, insoluble matters were filtered off by using Celite. The filtrate was washed with a saturated aqueous solution of ammonium chloride, and the organic layer was dried over anhydrous magnesium sulfate. The inorganic salt was separated by filtration, and the solvent was concentration in a vacuum. Then, the obtained residue was purified by silica gel column chromatography (development system, n-hexane:ethyl acetate=49:1). Thus, 31 mg (yield: 14%) of the title compound of Step 1 was obtained.
¹H-NMR (CDCl₃): 0.88 (3H, s), 0.91 (3H, s), 0.99 (3H, s), 1.01 (3H, s), 1.12 (3H, s), 1.16 (3H, s), 1.22 (3H, s), 1.38 (3H, s), 1.44 (3H, s), 0.82-2.19 (21H, m), 2.35 (1H, t, J=2.4 Hz), 3.18 (1H, dd, J=3.2, 6.6 Hz), 3.23 (1H, d, J=11.5 Hz), 3.46 (1H, dd, J=4.4, 9.3Hz), 4.04 (1H, d, J=11.5 Hz), 4.06 (1H, dd, J=2.4, 16.1 Hz), 4.19 (1H, dd, J=2.4, 16.1 Hz), 5.24 (1H, t-like).
MS FAB (m/z): 559 (M+Na)⁺

### (Step 2)

### 22β-(2-propynyloxy)olean-12-ene-3β,24-diol (Compound 2)

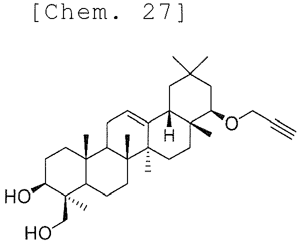

In a liquid mixture of 1.8 ml of chloroform and 1.8 ml of methanol, 179 mg of 22β-(2-propynyloxy)-3β,24-isopropylidenedioxyolean-12-ene was dissolved, and 90 µl of a 1 N aqueous solution of hydrochloric acid was added thereto, followed by stirring at room temperature for 20 minutes. The reaction was quenched with saturated aqueous sodium hydrogen carbonate. The reaction solution was diluted with chloroform, and then washed with saturated aqueous sodium chloride. After drying over anhydrous magnesium sulfate, the inorganic salt was separated by filtration, and the solvent was concentrated in a vacuum. The obtained residue was purified by silica gel column chromatography (development system, n-hexane:ethyl acetate=4:1 to 7:3). Thus, 125 mg (yield: 75%) of Compound 2 was obtained.
¹H-NMR (CDCl₃): 0.87 (3H, s), 0.89 (3H, s), 0.90 (3H, s), 0.94 (3H, s), 1.00 (3H, s), 1.11 (3H, s), 1.25 (3H, s), 0.83-1.88 (20H, m), 2.11 (1H, m), 2.34 (1H, dd, J=4.2, 11.0 Hz), 2.35 (1H, t, J=2.4 Hz), 2.68 (1H, m), 3.18 (1H dd, J=3.2, 6.8 Hz), 3.35 (1H, m), 3.44 (1H, m), 4.06 (1H, dd, J=2.4, 15.8 Hz), 4.17 (1H, dd, J=2.4, 15.8 Hz), 4.22 (1H, m), 5.22 (1H, t-like).
MS FAB (m/z): 519 (M+Na)⁺

### [Example 3]

### 22β-(4-hydroxymethylbenzyloxy)olean-12-ene-3β,24-diol (Compound 3)

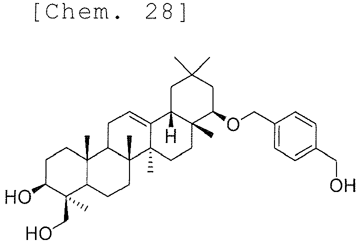

In 1.2 ml of methanol and 2.4 ml of THF, 152 mg of 22β-(4-formylbenzyloxy)-3β,24-isopropylidenedioxyolean -12-ene was dissolved, and 9.9 mg of sodium borohydride was added thereto under ice-cooling, followed by stirring for 10 minutes. After that, 1 ml of a 1 N aqueous solution of hydrochloric acid was added thereto, and the temperature was raised to room temperature, followed by stirring for 20 minutes. The reaction was quenched with saturated aqueous sodium hydrogen carbonate. Then, the reaction solution was diluted with chloroform, and washed with saturated aqueous sodium chloride. Extraction from the aqueous layer with chloroform was conducted twice, and then the combined organic layers were dried over anhydrous magnesium sulfate. The inorganic salt was separated by filtration, and the solvent was concentrated in a vacuum. Then, the obtained residue was purified by silica gel column chromatography (development system, n-hexane:ethyl acetate=3:2 to 1:1). Thus, 115 mg (yield: 80%) of Compound 3 was obtained.
¹H-NMR (CDCl₃) : 0.89 (6H, s), 0.93 (3H, s), 0.94 (3H, s), 1.04 (3H, s), 1.11 (3H, s), 1.25 (3H, s), 0.83-1.88 (21H, m), 2.15 (1H, m), 2.35 (1H dd, J=4.2, 11.0 Hz), 2.69 (1H m), 3.18 (1H, dd, J=2.9, 6.1 Hz), 3.35 (1H, m), 3.47 (1H, m), 4.20 (1H m), 4.30 (1H, d, J=11.9 Hz), 4.61 (1H, d, J=11.9 Hz), 4.68 (2H, d, J=6.1 Hz), 5.22 (1H, t-like), 7.33 (4H, s).
MS FAB (m/z): 601 (M+Na)⁺

### [Example 4] Method for producing 22β-(4-formylbenzyloxy)olean-12-ene-3β,24-diol (Compound 4)

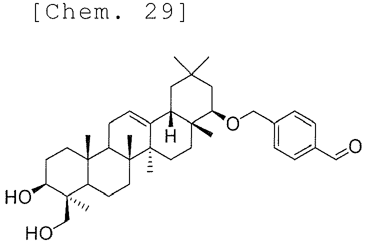

In 2.4 ml of THF, 148 mg of 22β-(4-formylbenzyloxy)-3β,24-isopropylidenedioxyolean -12-ene was dissolved, and 0.6 ml of a 1 N aqueous solution of hydrochloric acid was added thereto, followed by stirring at room temperature for 15 minutes. The reaction was quenched with saturated aqueous sodium hydrogen carbonate. The reaction solution was diluted with ethyl acetate, and then washed with saturated aqueous sodium chloride. After drying over anhydrous magnesium sulfate, the inorganic salt was separated by filtration, and the solvent was concentrated in a vacuum. The obtained residue was purified by silica gel column chromatography (development system, n-hexane:ethyl acetate=7:3 to 3:2). Thus, 130 mg (yield: 93%) of Compound 4 was obtained.
¹H-NMR (CDCl₃): 0.89 (3H, s), 0.90 (3H, s), 0.95 (6H, s), 1.04 (3H, s), 1. 12 (3H, s), 1.25 (3H, s), 0.83-1.88 (20H, m), 2.17 (1H, m), 2.35 (1H dd, J=4.2, 11.0 Hz), 2.68 (1H, m), 3.10 (1H, dd, J=2.9, 6.1 Hz), 3.35 (1H, m), 3.44 (1H, m), 4.20 (1H, m), 4.39 (1H, d, J=12.9 Hz), 4.70 (1H, d, J=12.9 Hz), 5.24 (1H, t-like), 7.50 (2H, d, J-8.1 Hz), 7. 84 (2H, d, J=8.1 Hz), 10.0 (1H, s).
MS FAB (m/z): 599 (M+Na)⁺

### [Example 5] Method for producing 22β-(2-hydroxypropoxy)olean-12-ene-3β,24-diol (Compound 5)

### (Step 1)

### 22β-(2-hydroxypropoxy)-3β24-isopropylidenedioxyolean-12-ene, and 22β3-hydroxypropoxy)-3β,24-isopropylidenedioxyolean-12-ene

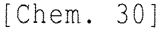

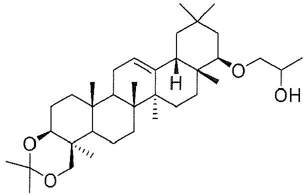

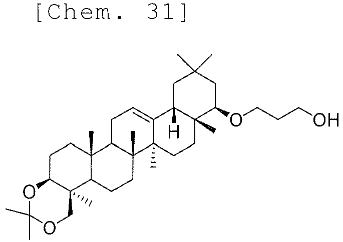

A solution of 1.36 g of 22β-allyloxy-3β,24-isopropylidenedioxyolean-12-ene (synthesized by a method described in DESCRIPTION of Japanese Patent No. 3279574) in 13.6 ml of a THF was cooled to -40°C, and then 2.5 ml of borane dimethyl sulfide complex (2.0 M tetrahydrofuran solution) was added thereto. The mixture was stirred for 30 minutes at the same temperature, and for 1 hour after the temperature was raised to room temperature. Under ice-cooling, 1.3ml of borane dimethyl sulfide complex (2.0 M tetrahydrofuran solution) was further added, followed by stirring at room temperature for 1.5 hours. After ice-cooling, water was slowly added until no further bubbles were generated. Then, 5 ml of a 5 N aqueous solution of sodium hydroxide and 9 ml of a 31% aqueous solution of hydrogen peroxide were added thereto, followed by stirring at room temperature for 1 hour. The reaction solution was diluted with ethyl acetate, then washed with saturated aqueous sodium hydrogen carbonate and saturated aqueous sodium chloride in this order, and dried over anhydrous sodium sulfate. The inorganic salt was separated by filtration, and the solvent was concentrated in a vacuum. Then, the obtained residue was purified by silica gel column chromatography (development system, n-hexane:ethyl acetate=49:1 to 4:1). Thus, 236 mg (yield: 16%) of 22β-(2-hydroxypropoxy)-3β,24-isopropylidenedioxyolean-12-ene and 871 mg (yield: 62%) of 22β-(3-hydroxypropoxy)-3β,24-isopropylidenedioxyolean-12-ene were obtained.

22β-(2-hydroxypropoxy)-3β,24-isopropylidenedioxy olean-12-ene (a 1:1 diastereomer mixture with the compound shown below)
¹H-NMR (CDCl₃): 0.83-2.38 (51H, m), 2.37 (0.5H, d, J=3.2 Hz), 2.47 (0.5H, d, J=2.8 Hz), 2.95-3.02 (1.5H, m) 3.21-3.27 (1.5H, m), 3.32 (0.5H, t, J=9.3 Hz), 3.45 (1H dd, J=4.6, 9.5 Hz), 3.54 (0.5H, dd, J=2.8, 9.1 Hz), 3.91 (1H, m), 4.05 (1H, d, J=11.5 Hz), 5.25 (1H, t-like).
MS FAB (m/z): 557 (M⁺+1) .

22β-(3-hydroxypropoxy)-3β,24-isopropylidenedioxy olean-12-ene
¹H-NMR (CDCl₃) 0.86 (3H, s), 0.90 (3H, s), 0.98 (3H, s), 1.00 (3H, s), 1.11 (3H, s), 1.15 (3H, s), 1.22 (3H, s), 1.38 (3H, s), 1.44 (3H, s), 0.82-2.09 (23H, m), 2.54 (1H, t, J=5.6 Hz), 2.94 (1H, dd, J=2.9, 6.6 Hz), 3.23 (1H, d, J=11.6 Hz), 3.40 (1H, m), 3.45 (1H, m), 3.72-3.80 (3H, m), 4.05 (1H, d, J=11.6 Hz), 5.24 (1H, t-like).
MS FAB (m/z): 557 (M⁺+1).

### (Step 2)

### 22β-(2-hydroxypropoxy)olean-12-ene-3β,24-diol (Compound 5)

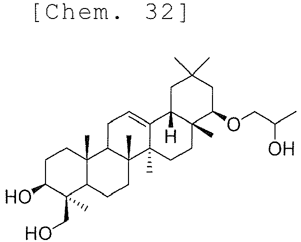

By using 225 mg of 22β-(2-hydroxypropoxy)-3β,24-isopropylidenedioxyolean-12-ene, 186 mg (89%) of Compound 5 was obtained by the same method as that in Step 2 of Example 2.
¹H-NMR (CDCl) : 0.83-1.88 (44H, m), 2.11-2.16 (1H, m), 2.37-2.48 (2H, m), 2.73 (0.5H, m), 2.95-3.01 (1.5H, m), 3.24 (0.5H, dd, J=3.2, 9.0 Hz), 3.22-3.38 (1.5H, m), 3.43-3.46 (1.5H, m), 3.54 (0.5H, dd, J=3.2, 9.0 Hz), 3.88-3.92 (1H, m), 4.19-4.22 (1H, m), 5.23 (1H, t-like).
MS ES (m/z): 517 (M⁺+1)

### [Example 6] Method for producing 22β-(3-phenoxypropoxy)olean-12-ene-3β,24-diol (Compound 6)

### (Step 1)

### 22β-(3-phenoxypropoxy)-3β,24-isopropylidenedioxyolean-12-ene

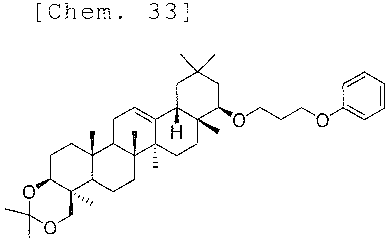

To a solution of 151 mg of 22β-(-hydroxypropoxy)-3β,24-isopropylidenedioxyolean-12-ene and 142 mg of triphenylphosphine in 2.7 ml of methylene chloride, 140 mg of 1,1'-(azodicarbonyl)dipiperidine and 52 mg of phenol were added under ice-cooling. After being stirred at room temperature for 1 hour, the reaction solution was purified by silica gel column chromatography (development system, n-hexane:ethyl acetate=49:1 to 97:3). Thus, 164 mg (yield: 95%) of the title compound of Step 1 was obtained.
¹H-NMR (CDCl₃: 0.86 (3H, s), 0.87 (3H, s), 0.98 (3H, s), 1.00 (3H, s), 1.12 (3H, s), 1.15 (3H, s), 1.22 (3H, s), 1.38 (3H, s), 1.44 (3H, s), 0.86-2.12 (23H, m), 2.91 (1H m), 3.23 (1H, d, J=11.5 Hz), 3.36 (1H dt, J=9.5, 5.8 Hz), 3.45 (1H, dd, J=4.6, 9.5 Hz), 3.70 (1H, dt, J=9.0, 5.8 Hz), 4.03-4.08 (3H, m), 5.24 (1H, t-like), 6.88-7.00 (3H, m), 7.25-7.29 (2H, m).
MS FAB (m/z): : 633 (M⁺+1).

### (Step 2)

### 22β-(3-phenoxypropoxy)olean-12-ene-3β,24-diol (Compound 6)

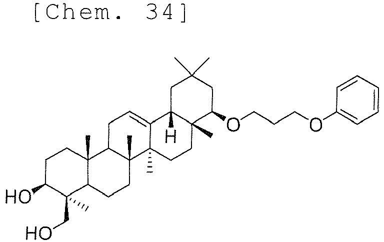

By using 176 mg of 22β-(3-phenoxypropoxy)-3β,24-isopropylidenedioxyolean-12-ene, 186 mg (yield: 98%) of Compound 6 was obtained by the same method as that in Step 2 of Example 2.
¹-NMR (CDCl₃: 0.86 (3H, s), 0.87 (3H, s), 0.89 (3H, s), 0.94 (3H, s), 1.00 (3H, s), 1.11 (3H, s), 1.25 (3H, s), 0.83-1.87 (20H, m), 1.98-2.09 (2H, m), 2.12 (1H, m), 2.35 (1H, m), 2.71 (1H, m), 2.91 (1H, m), 3.29-3.38 (2H, m), 3.44 (1H, m), 3.70 (1H, m), 4.07 (2H, t, J=6.3 Hz), 4.22 (1H, d, J=11.0 Hz), 5.21 (1H, t-like), 6.88-6.94 (2H, m), 7.25-7.29 (2H, m).
MS FAB (m/z): 631 (M+K)⁺

### [Example 7] Method for producing 22β-(3-hydroxypropoxy)olean-12-ene-3β,24-diol (Compound 7)

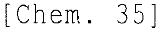

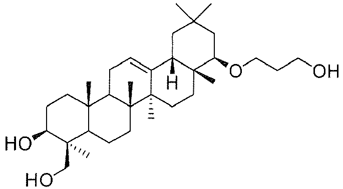

By using 151 mg of 22β-(3-hydroxypropoxy)-3β,24-isopropylidenedioxyolean-12-ene, 114 mg (81%) of Compound 7 was obtained by the same method as that in Step 2 of Example 2.
¹H-NMR (CDCl₃): 0.86 (3H, s), 0.89 (3H, s), 0.90 (3H, s), 0.93 (3H, s), 1.01 (3H, s), 1.11 (3H, s), 1.24 (3H, s), 0.83-1.91 (22H, m), 2.07 (1H, m), 2.64 (2H, brs), 2.88 (1H, brs), 2.94 (1H, dd, J=2.9, 6.6 Hz), 3.32-3.46 (3H, m), 3.72-3.79 (3H, m), 4.21 (1H, d, J=11.2 Hz), 5.21 (1H, t-like).
MS FAB (m/z): 516 (M⁺)

### [Example 8] Method for producing 22β-(3-(4-carboxyphenyl)oxypropoxy)olean-12-ene-3β,24-diol (Compound 8)

### (Step 1)

### 22β-(3-(4-methoxycarbonylphenyl)oxypropoxy)-3β,24-isop ropylidenedioxyolean-12-ene

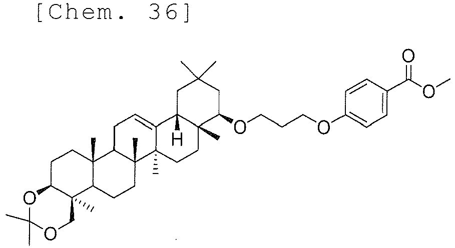

By using 181 mg of 22β-(3-hydroxypropoxy)-3β,24-isopropylidenedioxyolean-12-ene and 101 mg of 4-hydroxybenzoic acid methyl ester, 207 mg (yield: 92%) of the title compound of Step 1 was obtained by the same method as that in Step 1 of Example 6.
¹H-NMR (CDCl₃): 0.86 (3H, s), 0.87 (3H, s), 0.98 (3H, s), 0.99 (3H, s), 1.12 (3H, s), 1.15 (3H, s), 1.22 (3H, s), 1.38 (3H, s), 1.44 (3H, s), 0.85-2.12 (23H, m), 2.91 (1H, dd, J=2.9, 5.5 Hz), 3.23 (1H, d, J=11.5 Hz), 3.35 (1H, dt, J=9.4, 6.4 Hz), 3.45 (1H, dd, J=4.2, 9.0 Hz), 3.70 (1H, dt, J=9.4, 5.5 Hz), 3.88 (3H, s), 4.05 (1H, d, J=11.7 Hz), 4.09-4.15 (2H, m), 5.22 (1H, t-like), 6.8-7.26 (2H, m), 7.96-7.99 (2H, m).
MS FAB (m/z): 691 (M⁺+1).

### (Step 2)

### 22β-(3-(4-carboxyphenyl)oxypropoxy)olean-12-ene-3β,24-diol (Compound 8)

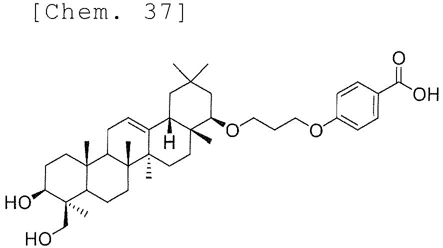

To a solution of 203 mg of 22β-(3-(4-methoxycarbonylphenyl)oxypropoxy)-3β,24-isop ropylidenedioxyolean-12-ene in 4 ml of THF, 1.6 ml of a 1 N aqueous solution of sodium hydroxide and 1.5 ml of methanol were added, followed by stirring at room temperature for 1 hour, and at 60°C for 3.5 hours. After cooling to room temperature, 0.63ml of a 5 N aqueous solution of hydrochloric acid was added thereto, followed by stirring at room temperature for 10 minutes. Then, the mixture was diluted with saturated aqueous sodium chloride. Extraction with ethyl acetate was conducted twice. Then, the combined organic layers were dried over anhydrous magnesium sulfate, and the inorganic salt was separated by filtration. The solvent was concentrated in a vacuum. Then, the obtained residue was purified by silica gel column chromatography (development system, methanol:chloroform=1:49). Thus, 177 mg (yield: 95%) of Compound 8 was obtained.
¹H-NMR (CDCl₃) : 0.85 (3H, s), 0.87 (3H, s), 0.89 (3H, s), 0.93 (3H, s), 0.99 (3H, s), 1.11 (3H, s), 1.25 (3H, s), 0.83-1.87 (20H, m), 2.00-2.04 (2H, m), 2.12 (1H, m), 2.91 (1H, m), 3.33-3.38 (2H, m), 3.44 (1H, m), 3.70 (1H, m), 4.13-4.16 (2H, m), 4.21 (1H, d, J=11.2Hz), 5.21 (1H, t-like), 6.92-6.94 (2H, m), 8.02-8.05 (2H, m).
MS ES (m/z): 637 (M⁺+1)

### [Example 9] Method for producing 22β-(4-(4-morpholylmethyl)benzyloxy)olean-12-ene-3β,24 -diol (Compound 9)

### (Step 1)

### 22β-(4-(4-morpholylmethyl)benzyloxy)-3β,24-isopropylid enedioxyolean-12-ene

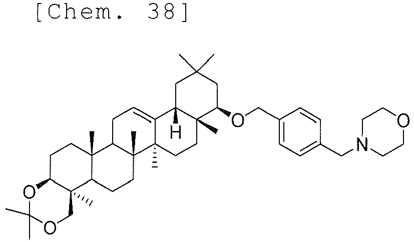

To a solution of 200 mg of 22β-(4-formylbenzyloxy)-3β,24-isopropylidenedioxyolean -12-ene in 6 ml of 1, 2-dichloroethane, 142 µl of morpholine, 74 µl of acetic acid, and 600 mg of anhydrous magnesium sulfate were added, followed by stirring at room temperature for 10 minutes. After ice-cooling, 139 mg of sodium triacetoxyborohydride was added, followed by stirring at room temperature overnight. After saturated aqueous sodium hydrogen carbonate and saturated aqueous sodium chloride were added thereto, extraction with chloroform was conducted three times. The combined organic layers were dried over anhydrous magnesium sulfate, and the inorganic salt was separated by filtration. The solvent was concentrated in a vacuum. Then, the obtained residue was purified by silica gel column chromatography (development system, methanol:chloroform=1:99 to 1:49). Thus, 223 mg (yield: 99%) of the title compound of Step 1 was obtained.
¹H-NMR (CDCl₃): 0.89 (3H, s), 0.93 (3H, s), 0.98 (3H, s), 1.04 (3H, s), 1.11 (3H, s), 1.15 (3H, s), 1.22 (3H, s), 1.38 (3H, s), 1.47 (3H, s), 0.86-2.01 (20H, m), 2.15 (1H, m), 2.43-2.45 (4H, m), 3.07 (1H, dd, J=2.9, 6.3 Hz), 3.23 (1H, d, J=11.5 Hz), 3.44 (1H, dd, J=4.4, 9.5 Hz), 3.48 (2H, s), 3.69-3.72 (4H, m), 4.05 (1H, d, J=11.5 Hz), 4.30 (1H, d, J=11.9 Hz), 4.59 (1H, d, J=11.9 Hz), 5.23 (1H, t-like), 7.26-7.37 (4H, m).
MS ES (m/z): 688 (M⁺)

### (Step 2)

### 22β-(4-(4-morpholylmethyl)benzyloxy)olean-12-ene-3β,24 -diol (Compound 9)

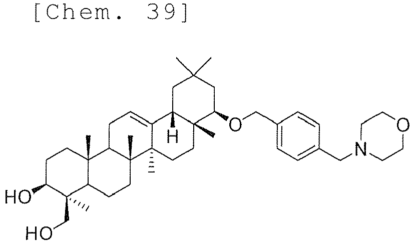

By using 220 mg of 22β-(4-(4-morpholylmethyl)benzyloxy)-3β,24-isopropylid enedioxyolean-12-ene, 189 mg (yield: 91%) of Compound 9 was obtained by the same method as that in Step 2 of Example 2.
¹H-NMR (CDCl₃): 0.89 (6H, s), 0.93 (3H, s), 0.94 (3H, s), 1.04 (3H, s), 1.11 (3H, s), 1.27 (3H, s), 0.83-1.86 (20H, m), 2.17 (1H, m), 2.42-2.44 (5H, m), 2.71 (1H, m), 3.06 (1H, dd, J=2.7, 6.1 Hz), 3.33 (1H, m), 3.43 (1H, m), 3.48 (2H, s), 3.69-3.72 (4H, m), 4.20 (1H, d, J=11.2 Hz), 4.30 (1H, d, J=11.7 Hz), 4.59 (1H, d, J=11.7Hz), 5.22 (1H, t-like), 7.23-7.30 (4H, m).
MS ES (m/z): 648 (M⁺+1)

### [Example 10] Method for producing 22β-(4-dimethylaminomethylbenzyloxy)olean-12-ene-3β,24 -diol (Compound 10)

### (Step 1)

### 22β-(4-dimethylaminomethylbenzyloxy)-3β,24-isopropylid enedioxyolean-12-ene

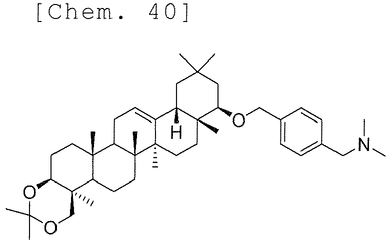

By using 200 mg of 22β-(4-formylbenzyloxy)-3β,24-isopropylidenedioxyolean -12-ene and 1. 6 ml of dimethylamine (2.0 M, THF solution), 204 mg (97%) of the title compound of Step 1 was obtained by the same method as that in Step 1 of Example 9.
¹H-NMR (CDCl₃): 0.89 (3H, s), 0.93 (3H, s), 0.98 (3H, s), 1.04 (3H, s), 1.11 (3H, s), 1.15 (3H, s), 1.22 (3H, s), 1.38 (3H, s), 1.44 (3H, s), 0.83-2.04 (20H, m), 2.13 (1H, m), 2.23 (6H, s), 3.06 (1H, dd, J=2.9, 6.3 Hz), 3.23 (1H, d, J=11.6 Hz), 3.41 (2H, s), 3.46 (1H, dd, J=4.4, 9.3 Hz), 4.05 (1H, d, J=11.6 Hz), 4.31 (1H, d, J=11.9 Hz), 4.59 (1H, d, J=11.9 Hz), 5.24 (1H, t-like), 7.26-7.53 (4H, m).
MS FAB (m/z): 646 (M⁺)

### (Step 2)

### 22β-(4-dimethylaminomethylbenzyloxy)olean-12-ene-3β,24 -diol (Compound 10)

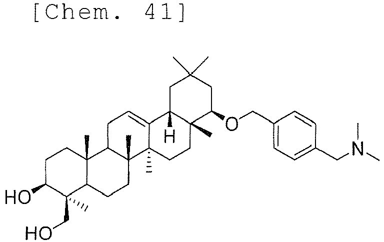

By using 200 mg of 22β-(4-dimethylaminomethylbenzyloxy)-3β,24-isopropylid enedioxyolean-12-ene, 128 mg (yield: 68%) of Compound 10 was obtained by the same method as that in Step 2 of Example 2.
¹H-NMR (CDCl₃): 0.89 (6H, s), 0.92 (3H, s), 0.94 (3H, s), 1.04 (3H, s), 1.10 (3H, s), 1.24 (3H, s), 0.83-1.86 (20H, m), 2.17 (1H, m), 2.23 (6H, s), 3.06 (1H, dd, J=2.9, 6.3 Hz), 3.34 (1H, d, J=11.2 Hz), 3.40 (2H, s), 3.45 (1H, dd, J=4.2, 11.5Hz), 4.20 (1H, d, J=11.2Hz), 4.31 (1H, d, J=11.9 Hz), 4.59 (1H d, J=11.9 Hz), 5.24 (1H, t-like), 7.25-7.29 (4H, m).
MS ES (m/z): 606 (M⁺+1)

### [Example 11] Method for producing 22β-(4-phenylaminomethylbenzyloxy)olean-12-ene-3β,24-d iol (Compound 11)

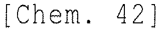

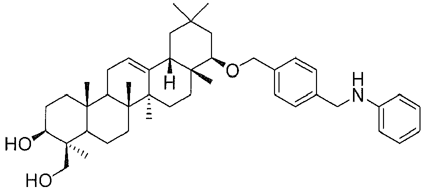

By using 340 mg of 22β-(4-formylbenzyloxy)-3β,24-isopropylidenedioxyolean -12-ene and 0.25 ml of aniline, a reductive amination reaction was conducted by the same method as that in Step 1 of Example 9, and then a deprotection reaction was conducted by the same method as that in Step 2 of Example 2. Thus, 304 mg (yield: 84%, 2 steps) of Compound 11 was obtained.
¹H-NMR (CDCl₃): 0.89 (6H, s), 0.93 (3H, s), 0.94 (3H, s), 1.04 (3H, s), 1.11 (3H, s), 1.25 (3H, s), 0.83-1.86 (20H, m), 2.05 (1H, m), 2.41 (1H, m), 2.72 (1H, m), 3.06 (1H, dd, J=2.9, 5.8 Hz), 3.34 (1H, m), 3.44 (1H, m), 4.03 (1H, m), 4.22 (1H, d, J=11.0 Hz), 4.30 (1H, d, J=12.0 Hz), 4.31 (2H, s), 4.59 (1H, d, J=12.0 Hz), 5.22 (1H, t-like), 6.62-6.65 (2H, m), 6.71 (1H, m), 7.14-7.20 (2H, m), 7.29-7.34 (4H, m).
MS ES (m/z): 654 (M⁺+1)

### [Example 12] Method for producing 22β-benzyloxyolean-12-ene-3β-hydroxy-24-oic acid (Compound 12)

### (Step 1)

### 22β-benzyloxy-3-oxoolean-12-en-24-oic acid methyl ester

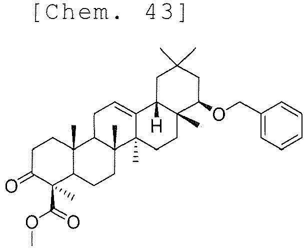

To a solution of 504 mg of 22β-benzyloxy-3β,24-isopropylidenedioxyolean-12-ene (synthesized by a method described in DESCRIPTION of Japanese Patent No. 3279574) in 5 ml of chloroform and 5 ml of methanol, 0.5 ml of a 1 N aqueous solution of hydrochloric acid was added, followed by stirring at room temperature for 30 minutes. The reaction was quenched with saturated aqueous sodium hydrogen carbonate. Then, the reaction solution was diluted with saturated aqueous sodium chloride, and extraction with chloroform was conducted three times. The combined organic layers were dried over anhydrous magnesium sulfate, then the inorganic salt was separated by filtration, and the solvent was concentrated in a vacuum.

The obtained residue was dissolved in 8.5 ml of methylene chloride, and 521 mg of molecular sieves 4Å, 408 mg of 4-methylmorpholine N-oxide, and 30 mg of tetra-N-propylammonium perruthenate were added thereto, followed by stirring at room temperature for 20 minutes. The reaction solution was directly subjected to purification by silica gel column chromatography (development system, n-hexane:ethyl acetate=49:1). Thus, an aldehyde compound was obtained. The aldehyde compound was directly used in the following reaction.

The aldehyde was dissolved in 4.3 ml of THF and 4.3 ml of 2-methylpropanol, and then 0.45 ml of 2-methyl-2-butene was added thereto. A solution of 210 mg of sodium dihydrogen phosphate dihydrate and 147 mg of sodium chlorite in 2.1 ml of water was added thereto, followed by stirring at room temperature for 40 minutes. Further, a solution of 100 mg of sodium dihydrogen phosphate dihydrate and 72 mg of sodium chlorite in 1 ml of water was added thereto, followed by stirring at room temperature for 20 minutes. The reaction was quenched with a saturated aqueous solution of sodium thiosulfate, and the reaction solution was diluted with dilute hydrochloric acid and ethyl acetate. After liquid separation, the organic layer was washed with water and saturated aqueous sodium chloride, and dried with an aqueous solution of anhydrous magnesium sulfate. The inorganic salt was separated by filtration, and then the solvent was concentrated in a vacuum. The obtained residue was purified by silica gel column chromatography (development system, methanol:chloroform=1:99 to 1:45). Thus, a carboxylic acid compound was obtained. The carboxylic acid compound was directly used in the following reaction.

To a solution of the carboxylic acid in 8 ml of toluene and 2 ml of methanol, 2.1 ml of trimethylsilyldiazomethane (0.6 M, hexane solution) was added, followed by stirring at room temperature for 10 minutes. The solvent was concentrated in a vacuum, then purification by silica gel column chromatography was conducted (development system, n-hexane:ethyl acetate=99:1 to 97:3). Thus, 361 mg (yield: 73%, 4 steps) of the title compound of Step 1 was obtained.
¹H-NMR (CDCl₃) : 0.89 (3H, s), 0.94 (3H, s), 1.04 (6H, s), 1.07 (3H, s), 1.10 (3H, s), 1.37 (3H, s), 0.89-2.05 (18H, m), 2.17 (1H, m), 2.36 (1H, m), 2.95 (1H, ddd, J=6.3,14.6, 14.6 Hz), 3.08 (1H, dd, J=2.7, 6.3 Hz), 3.68 (3H, s), 4.32 (1H, d, J=11.7Hz), 4.62 (1H, J=11.7Hz), 5.24 (1H, t-like), 7.24-7.35 (5H, m).
MS FAB (m/z): 575 (M⁺+1)

### (Step 2)

### 22β-benzyloxyolean-12-ene-3β-hydroxy-24-oic acid methyl ester

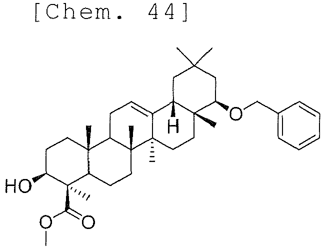

A solution of 238 mg of 22β-benzyloxy-3-oxoolean-12-en-24-oic acid methyl ester in 4.1 ml of THF and 1 ml of methanol was cooled to -78 °C, and then 33 mg of sodium borohydride was added. The temperature was raised to 0°C over 1.5 hours, and then the reaction was quenched with a saturated aqueous solution of ammonium chloride. The reaction solution was diluted with water. Extraction with ethyl acetate was conducted, and then the organic layer was washed with saturated aqueous sodium chloride, and dried over anhydrous magnesium sulfate. The inorganic salt was separated by filtration, and then the solvent was concentrated in a vacuum. The obtained residue was purified by silica gel column chromatography (development system, n-hexane: ethyl acetate=19: 1 to 9:1). Thus, 228 mg (yield: 95%) of the title compound of Step 2 was obtained.
¹H-NMR (CDCl₃): 0.79 (3H, s), 0.89 (3H, s), 0.94 (3H, s), 0.98 (3H, s), 1.04 (3H, s), 1.11 (3H, s), 1.40 (3H, s), 0.83-1.89 (19H, m), 2.03 (1H, m), 2.15 (1H, m), 3.06-3.12 (2H, m), 3.35 (1H, d, J=12.0 Hz), 3.68 (3H, s), 4.32 (1H, d, J=12. 0 Hz) , 4.62 (1H, d, J=12. 0 Hz) , 5.24 (1H, t-like), 7.23-7.35 (5H, m).
MS FAB (m/z): 577 (M⁺+1)

### (Step 3)

### 22β-benzyloxyolean-12-ene-3β-hydroxy-24-oic acid (Compound 12)

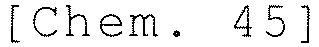

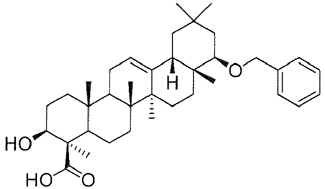

To 9.5 ml of potassium tert-butoxide (1.0 M, THF solution), 42.4 µl of water and a solution of 218 mg of 22β-benzyloxyolean-12-en-3β-ol-24-oic acid methyl ester in 2 ml of THF were added in this order. Then, a reaction was conducted at 75°C for 1 hour by using a microwave reactor. After cooling to room temperature, adjustment to an acidic state was conducted by using water and a 5 N aqueous solution of hydrochloric acid, and extraction was conducted with ethyl acetate. The organic layer was washed with saturated aqueous sodium chloride, and then dried over anhydrous magnesium sulfate, and the inorganic salt was separated by filtration. The solvent was concentrated in a vacuum. Then, the obtained residue was purified by preparative TLC (development system, methanol:chloroform=3:97). Thus, 112 mg (yield: 52%) of Compound 12 was obtained.
¹H-NMR (CDCl₃) : 0.90 (3H, s), 0.91 (3H, s), 0.94 (3H, s), 1.00 (3H, s), 1.04 (3H, s), 1.11 (3H, s), 1.48 (3H, s), 0.85-1.90 (19H, m), 2.00 (1H, m), 2.15 (1H, m), 3.08 (1H, m), 3.15 (1H, dd, J=4.2,12.1), 4.32 (1H, d, J=12.0 Hz), 4.62 (1H, d, J=12.0 Hz), 5.24 (1H, m), 7.26-7.35 (5H, m).
MS ES (m/z): 563 (M⁺+1)

### [Example 13] Method for producing 22β-allyloxyolean-12-ene-3β-hydroxy-24-oic acid (Compound 13)

### (Step 1)

### 22β-allyloxy-3-oxoolean-12-en-24-oic acid methyl ester

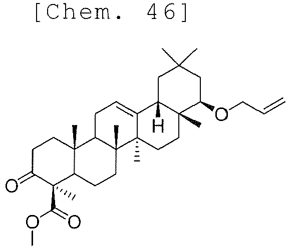

By using 817 mg of 22β-allyloxy-3β,24-isopropylidenedioxyolean-12-ene (synthesized by a method described in DESCRIPTION of Japanese Patent No. 3279574), 559 mg (70%, 4 steps) of the title compound of Step 1 was obtained by the same method as that in Step 1 of Example 12.
¹H-NMR (CDCl₃): 0.89 (3H, s), 0.90 (3H, s), 1.01 (3H, s), 1.04 (3H, s), 1.07 (3H, s), 1.10 (3H, s), 1.37 (3H, s), 0.86-2.05 (18H, m), 2.12 (1H, m), 2.38 (1H, ddd, J=2.3,4.6, 14.6 Hz), 2.90-3.00 (2H, m), 3.68 (3H, s), 3.80 (1H, m), 4.05 (1H, m), 5.11 (1H, m), 5.23-5.29 (2H, m), 5.90 (1H, m).
MS FAB (m/z): 525 (M⁺+1)

### (Step 2)

### 22β-allyloxyolean-12-ene-3β-hydroxy-24-oic acid methyl ester

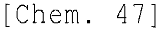

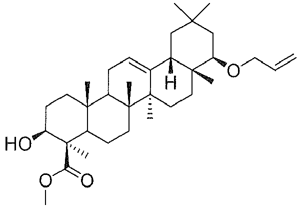

By using 557 mg of 22β-allyloxy-3-oxoolean-12-en-24-oic acid methyl ester, 551 mg (98%) of the title compound of Step 2 was obtained by the same method as that in Step 2 of Example 12.
¹H-NMR (CDCl₃): 0.79 (3H, s), 0.89 (6H, s), 0.98 (3H, s), 1.01 (3H, s), 1.11 (3H, s), 1.41 (3H, s), 0.82-1.89 (19H, m), 1.99 (1H, m), 2.09 (1H, m), 2.99 (1H, dd, J=2.9, 6.8 Hz), 3.09 (1H, dt, 4.4, 12.0 Hz), 3.35 (1H d, J=12.0 Hz), 3.68 (3H, s), 3.79 (1H, m), 4.06 (1H m), 5.11 (1H, m), 5.23-5.28 (2H, m), 5.90 (1H, m).
MS FAB (m/z): 527 (M⁺+1)

### (Step 3)

### 22β-allyloxyolean-12-ene-3β-hydroxy-24-oic acid (Compound 13)

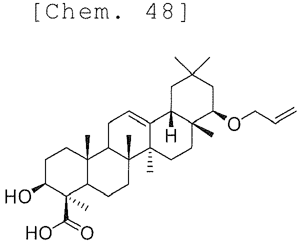

By using 550 mg of 22β-allyloxyolean-12-en-3β-ol-24-oic acid methyl ester, 321 mg (60%) of compound 13 was obtained by the same method as that in Step 3 of Example 12.
¹H-NMR (CDCl₃): 0.89 (6H, s), 0.90 (3H, s), 1.00 (3H, s), 1.01 (3H, s), 1.12 (3H, s), 1.48 (3H, s), 0.85-1.90 (19H, m), 2.01 (1H, m), 2.12 (1H, m), 2.99 (1H dd, J=2.9, 6.8 Hz), 3.15 (1H, dd, J=4.2,11.7), 3.80 (1H, dd, J=5.4, 13.0 Hz), 4.06 (1H, dd, J=5.1, 13.0 Hz), 5.10 (1H, dd, J=1.5, 10.5 Hz), 5.23-5.28 (2H, m), 5.90 (1H, m).
MS ES (m/z): 535 (M+Na)⁺ +

### [Example 14] Method for producing 3β-hydroxy-22β-methoxyolean-12-en-24-oic acid (Compound 14)

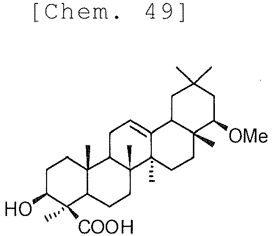

In 200 ml of pyridine, 20.0 g of 22β-methoxyolean-12-ene-3β,24-diol (synthesized by a method described in DESCRIPTION of Japanese Patent No. 3279574) was dissolved, and 17.5 g of triphenylmethyl chloride was added thereto, followed by stirring at 75°C for 24 hours. After cooling to room temperature, the solvent was evaporated under reduced pressure. Then, water and 350 ml of ethyl acetate were added to the residue, and liquid separation was conducted. The organic layer was washed with saturated aqueous sodium chloride, and dried over anhydrous sodium sulfate. Then, the inorganic salt was separated by filtration, and the solvent was concentrated in a vacuum. The obtained residue was dissolved in 450 ml of DMF, and heated to 45°C. To the solution, 5.0 g of 60% sodium hydride was added, followed by stirring for 1 hour. Then, 22 g of benzyl bromide was added thereto, followed by stirring for 16 hours. After cooling to room temperature, 2 L of ethyl acetate and 3 L of water were added thereto, and liquid separation was conducted. The organic layer was washed with saturated aqueous sodium chloride, and dried over anhydrous sodium sulfate. Then, the inorganic salt was separated by filtration, and the solvent was concentrated in a vacuum. The obtained residue was purified by silica gel column chromatography (development system, benzene:n-hexane=2:3). Thus, 9.1 g of 3β-benzyloxy-22β-methoxyolean-24-triphenylmethyloxy-12 -ene was obtained. In a liquid mixture of 32 ml of acetone and 190 ml of methanol, 9.1 g of 3β-benzyloxy-22β-methoxyolean-24-triphenylmethyloxy-12 -ene was dissolved, and 32 ml of concentrated hydrochloric acid was added thereto, followed by heating under reflux for 2.5 hours. The reaction solution was cooled, and neutralized with a 2 N aqueous solution of sodium hydroxide. Then, the solvent was evaporated. To the residue, 700 ml of ethyl acetate and water was added, and liquid separation was conducted. The organic layer was washed with saturated aqueous sodium chloride, and dried over anhydrous sodium sulfate. Then, the inorganic salt was separated by filtration, and the solvent was concentrated in a vacuum. The obtained residue was purified by silica gel column chromatography (development system, benzene:n-hexane=20:1). Thus, 2.7 g of 3β-benzyloxy-22β-methoxyolean-12-en-24-ol was obtained.

In 80 ml of methylene chloride, 3.6 ml of oxalyl chloride was dissolved, and the solution was cooled to -65°C, followed by stirring for 10 minutes. A solution of 2.7 g of 3β-benzyloxy-22β-methoxyolean-12-en-24-ol dissolved in 23 ml of methylene chloride was added thereto, followed by stirring further for 1.5 hours. To the reaction solution, 16 ml of triethylamine was added, and the reaction solution was stirred, while the temperature was being raised to room temperature. After 50 ml of water was added, extraction with 40 ml of methylene chloride was conducted. The organic layer was washed with a saturated aqueous solution of sodium hydrogen carbonate, and dried over anhydrous sodium sulfate. After that, the inorganic salt was separated by filtration, and the solvent was concentrated in a vacuum. The obtained residue was dissolved in 150 ml of t-butanol, and 16.7 g of 2-methyl-2-butene was added thereto. Then, the mixture was heated to 30°C. To this reaction liquid, a solution of 2.9 g of sodium dihydrogen phosphate and 2.7 g of sodium chlorite dissolved in 15 ml of water was added stepwise in three divided portions, followed by stirring at 30°C for 2 days. The t-butanol was evaporated under reduced pressure, and the residue was dissolved in ethyl acetate. Then, the solution was washed with saturated aqueous sodium chloride, and dried over anhydrous sodium sulfate. After that, the inorganic salt was separated by filtration, and the solvent was concentrated in a vacuum. The obtained residue was purified by silica gel column chromatography (development system, hexane:ethyl acetate=7:1). Thus, 2.7 g of 3β-benzyloxy-22β-methoxyolean-12-en-24-oic acid was obtained. In a liquid mixture of 70 ml of ethyl acetate and 45 ml of ethanol, 2.7 g of 3β-benzyloxy-22β-methoxyolean-12-en-24-oic acid was dissolved, and 0.90 g of palladium hydroxide/C was added thereto, followed by stirring under a hydrogen gas atmosphere at room temperature for 4 hours. The catalyst was separated by filtration using Celite. Then, the solvent was concentrated, and ethyl acetate was added to the obtained residue. The precipitated solid was collected by filtration. Thus, 0.85 g (yield: 4%) of Compound 14 was obtained.
¹H-NMR (CDCl₃) : 0.86 (3H, s), 0.90 (6H, s), 1.00 (6H, s), 1.11 (3H, s), 1.31 (1H, dd, J=3.0, 13.9 Hz), 1.48 (3H, s), 0.88-2.12 (20H, m), 2.82 (1H, dd, J=2.8, 7.0 Hz), 3.15 (1H, dd, J=4.4, 12.4 Hz), 3.28 (3H, s), 5.24 (1H, dd, J=3.6, 3.6 Hz).
MS FAB (m/z): 509 (M⁺+Na)

### [Example 15] Method for producing 3β-hydroxy-22β-methoxyolean-12-en-24-yl sulfate sodium salt (Compound 15)

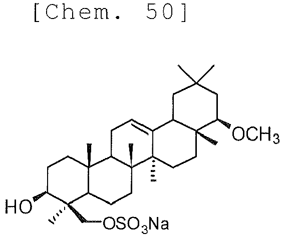

In 200 ml of dioxane, 10.0 g of 3β-benzyloxy-22β-methoxyolean-12-en-24-ol (synthesized by the method described in Example 14) was dissolved, and a solution of 6.2 g of chlorosulfonic acid dissolved in 40 ml of dioxane was added dropwise at 12 °C thereto. After stirring at room temperature for 1 hour, 40 ml of cold water was added thereto, followed by concentration in a vacuum. The obtained residue was dissolved in 200 ml of ethanol, and 1 g of palladium hydroxide/C was added thereto, folllowed by sttiring at room temperature for 1 hour, with hydrogen gas being introduced. The palladium catalyst was separated by filtration using Celite, and the solvent was concentrated in a vacuum. The obtained residue was purified by silica gel column chromatography (development system, chloroform:methanol=5:1). The elution fraction was made alkaline by adding a 28% methanol solution of sodium methoxide, and then concentrated in a vacuum. Thus, crude crystals were obtained. The obtained crude crystals were washed with water, and collected by filtration. The collected crystals were dissolved in a mixture solution of methanol and THF. After insoluble matters were separated by filtration, the mixture was concentrated in a vacuum. The obtained crystals were subjected to slurry washing with ethyl acetate, and then dried under a reduced pressure. Thus, 3.35g (33%) of Compound 15 was obtasined.
¹H-NMR (DMSO-d6): 0.81 (3H, s), 0.87 (3H, s), 0.91 (3H, s), 0.94 (3H, s), 0.97 (3H, s), 1.01 (3H, s), 1.08 (3H, s), 0.68-1.89 (20H, m), 2.01-2.05 (1H, m), 2.79-2.81 (1H, m), 3.01-3.06 (1H, m), 3.19 (3H, s), 3.83 (1H, d, J=10.4 Hz), 3.86 (1H, d, J=10.4 Hz), 4.35 (1H, d, J=6.3 Hz), 5.17 (1H, t-like).
MS TSP (m/z): 551 (M⁺-1)

### [Example 16] Method for producing 1-O-(3β-hydroxy-22β-methoxyolean-12-en-24-yl)-β-D-gluc uronic acid (Compound 16)

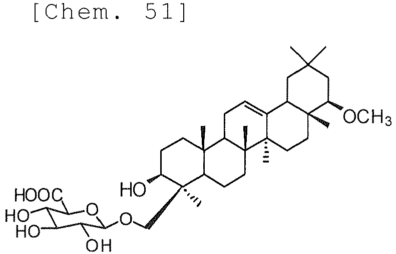

In 140 ml of benzene, 1.48 g of 3β-benzyloxy-22β-methoxyolean-12-en-24-ol was dissolved, and 3.78 g of calcium sulfate, 1.53 g of silver carbonate, and 1.83 g of 2,3,4-tri-O-acetyl-1-bromo-1-deoxy-α-D-glucuronic acid methyl ester were added thereto, followed by heating under reflux at 78 °C for 7 hours. After cooling to room temperature, insoluble matters were separated by filtration. Then, the liquid was concentrated in a vacuum, and 7.23 g of the obtained residue was purifed by silica gel column chromatography (development system, n-hexane:ethyl acetate=3:1). Thus, 1.08 g of 2,3,4-tri-O-acetyl-1-O-(3β-benzyloxy-22β-methoxyolean-12-en-24-yl)-β-D-glucuronic acid methyl ester was obtained.

In a mixture solvent of 25 ml of ethyl acetate and 16 ml of ethanol, 1.08 g of 2,3,4-tri-O-acetyl-1-O-(3β-benzyloxy-22β-methoxyolean-12-en-24-yl)-β-D-glucuronic acid methyl ester was dissolved, and 0.2 g of palladium hydroxide/C was added, followed by stirring under a hydrogen atmosphere at room temperature for 3 hours. The catalyst was separated by filtration, and the filtrate was concentrated in a vacuum. The obtained residue was purified by silica gel column chromatography (development system, n-hexane:ethyl acetate=2:1). Thus, 0.91 g of a debenzylation product was obtained.

In 140 ml of methanol, 0.91 g of the debenzylation product was dissolved, and 17.4 ml of a 2 N aqueous solution of potassium hydroxide was added, folowed by stirring at 60 °C for 8 hours. After cooled to room temperature, the mixture was neutralized by adding acetic acid, and then concentrated in a vacuum. Water was added to the obtained residue, and the precipitated crystals were collected by filtration. The obtained crystals were subjected to slurry washing with ethyl acetate and with THF. Thus, 517 mg of Compound 16 was obtained.
¹H-NMR (CD₃OD): 0.85 (3H, s), 0.89 (3H, s), 0.97 (3H, s), 0.98 (3H, s), 1.00 (3H, s), 1.14 (3H, s), 1.20 (3H, s), 0.81-1.91 (20H, m), 2.09 (1H, d, J=10.5 Hz), 2.84 (1H, dd, J=2.9, 6.1 Hz), 3.17-3.23 (2H, m), 3.26 (3H, s), 3.37 (1H, dd, J=9.0, 9.0 Hz), 3.48 (1H, dd, J=9.0, 9.0 Hz), 3.68 (1H, d, J=9.8 Hz), 3.79 (1H, d, J=9.8 Hz), 4.04 (1H, d, J=9.5 Hz), 4.28 (1H, d, J=7.8 Hz), 5.21 (1H, t-like).
MS TSP (m/z): 647 (M⁺-1)

### [Example 17] HCV (Con1 strain) Replicon Assay and Cytotoxicity Test

The compounds represented by the formula (I) were subjected to an HCV replicon assay and a cytotoxicity test.

In order to quantify the degree of HCV virus proliferation, a material was constructed by introducing a luciferase gene as a reporter gene into the genomic gene sequence of HCV Con1 strain. The luciferase gene was introduced in the form of fusion with a neomycin-resistant gene, immediately after the IRES (Internal Ribosome Entry Site) of the HCV gene according to the method of Krieger et al., (J. Virol. 75: 4614 (2001)). The constructed RNA was introduced into Huh7 cells by electroporation, and isolated as G418 resistant clone.

The HCV replicon cells comprising the luciferase gene and the neomycin resistance gene were suspended in a culture liquid [D-MEM (SIGMA, D6046) containig 10% of fetal bovine serum (EQUITECH-BIO, INC.)], and seeded onto 96-well plates at 5000 to 8100 cells/well. Test compounds were added at various concentrations, and the cells were cultured under 5%CO₂ at 37 °C for 3 days. For adding the test compounds, solutions of the test compounds dissolved in dimethyl sulfoxide (DMSO) were used, and diluted with the culture liquid to appropriate concentrations before use. The experiment was conducted with the final concentrations of DMSO used for dissolving the test compounds being 0.4% during the culturing of the replicon cells. To wells to which no test compound was added, DMSO was added with a final concentration of 0.4%.

Assay plates were prepared for two sytstems; one of the assays was conducted on a white plate, and the other was conducted on a clear plate. After the culturing for treatments with the test compounds was completed, the white plate was subjected to chemiluminescence measurement by usingaSteady-GloLuciferaseAssaySystem (Promega, E2520) in order to detect effects on HCV replicon proliferation. A EnVision multilabel counter (PerkinElmer 2102) was used for the chemiluminescence measurment. While the value obtained when no test compound was added was taken as 0% inhibition, the inhibitory ratios by the treatments with the agents were culculated.

In addition, evaluation of cytotoxicity of the treatments with the agents was conducted by using Cell Counting Kit-8 (Dojindo Laboratories, CK04). For measuring the cytotoxicity, the clear plate was used, and the measuremet was conducted after the culturing for treatments with the test compounds was completed, as in the case with the HCV replicon assay.

Regarding the HCV replicon assay, IC50s (50% growth inhibitory concentrations) of the agents were culculated, with the value obtained when no agent was added being taken as 0% inhibition. As a result, the IC50 value of Compound 17 was >20 µM, whereas the IC50 values of the compounds of the present invention were as shown in Table 3 below.

From these results, it was shown that the compounds of the present invention exhibited better HCV proliferation inhibition effect even than 22β-methoxyolean-12-ene-3β,24-diol (Compound 17).

**[Table 3]**

| Compound NO. | IC50 (50% inhibitory concentration) (µM) | Value growth |
|---|---|---|
| 17 | >20 | |
| | | |
| 1 | 13.49 | |
| 2 | 5.73 | |
| 3 | 1.56 | |
| 4 | 3.59 | |
| 7 | 5.32 | |
| 8 | 6.99 | |
| 9 | 9.13 | |
| 10 | 3.68 | |
| 11 | 12.95 | |
| 12 | 5.54 | |
| 13 | 7.05 | |
| 14 | 5.57 | |

### [Example 18] Pharmacokinetics Study in Mice

Balb/c mice (male, 7 to 8-week old) were used (3 or more mice per group). Each test compound suspended in olive oil was orally administered at 20 mg/kg, and blood was collected from the jugular vein after 2, 6, and 24 hours, or after 2, 4, 6, 8, 24, and 48 hours. The plasma drug concentration was measured by using a liquid chromatograph/mass spectrometry/mass spectrometry (LC-MS/MS). In addition, pharmacokinetics parameters were obtained from change in the obtained plasma concentration.

Table 4 shows the maximum plasma concentration (Cₘₐₓ), the plasma half-life (t_{1/2}), and the area under the plasma drug concentration-time curve (AUC) of each compound obtained in this study.

From the above-described results of the study on the oral administration to the mice, it was shown that the compounds of the present invention have excellent pharmacokinetics in terms of the high absorbability and the long-lasting plasma concentration, even when compared with 22β-methoxyolean-12-ene-3β,24-diol (Compound 17).

**[Table 4]**

| Compound No. | Cₘₐₓ (µg/ml) | t_{1/2} (hr) | AUC (µg·hr/ml) |
|---|---|---|---|
| 17 | 2.6±0.7 | 7.4±0.4 | 35.6±9.7 |
| | | | |
| 2 | 2.8±1.0 | 10.0±1.2 | 44.7±16.3 |
| 12 | 4.8±1.1 | 11.9±2.7 | 74.7±11.0 |
| 13 | 4.8±1.5 | 13.0±5.5 | 74.9±16.3 |
| 14 | 4.8±1.1 | 13.9±1.0 | 81.1±18.3 |

### [Industrial Applicability]

The triterpene derivative of the present invention can exhibit an excellent anti-HCV activity and excellent biokinetics, when administered in an effective amount. Hence, the triterpene derivative can be expected to provide a high prophylactic effect or a high therapeutic effect against chronic hepatitis C. Accordingly, the present invention is applicable, in particular, in the medical field.

## Claims

1. A triterpene derivative represented by a general formula (I) or apharmaceuticallyacceptable salt thereof: 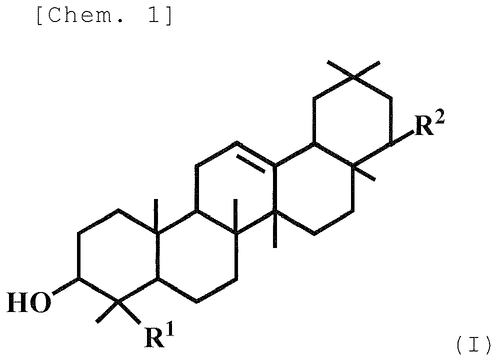 [in the formula (I), R¹ represents a carboxyl group, a hydroxymethyl group, -CH₂OSO₃H, or 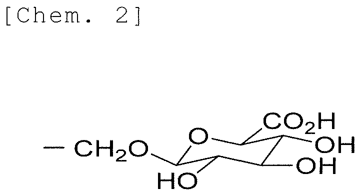 , and R² represents -OR³ or -O- (CH₂)ₘ-OR⁴, where R³ represents a benzyl group which may be substituted with a hydroxymethyl group, a dimethylaminomethyl group, a phenylaminomethyl group, a morpholinomethyl group, a carboxyl group, or a formyl group, a C₁₋₆ alkyl group, a C₂₋₆ alkenyl group, a C₂₋₆ alkynyl group, or a hydroxy C₁₋₆ alkyl group, R⁴ represents a phenyl group which may be substituted with a carboxyl group, and m represents an integer of 1 to 3, with the proviso that a case where R¹ is a hydroxymethyl group, R² is -OR³, and R³ is a C₁₋₆ alkyl group, a C₂₋₆ alkenyl group, or a benzyl group is excluded].

2. A triterpene derivative represented by a general formula (I) or a pharmaceutically acceptable salt thereof: 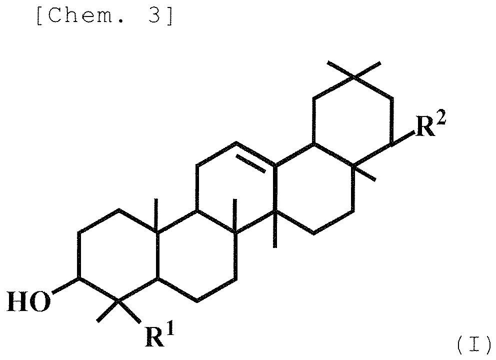 [in the formula (I), R¹ represents a carboxyl group, and R² represents -OR³, where R³ represents a benzyl group, a methyl group, or an allyl group].

3. A triterpene derivative represented by a general formula (I) or a pharmaceutically acceptable salt thereof: 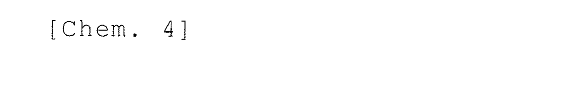 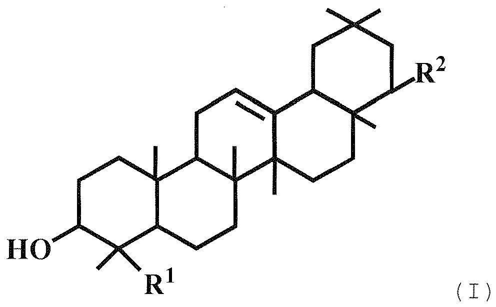 [in the formula (I), R¹ represents a hydroxymethyl group, and R² represents -OR³ or -O-(CH₂)₃-OR⁴, where R³ represents a benzyl group substituted with a hydroxymethyl group, a dimethylaminomethyl group, a phenylaminomethyl group, a morpholinomethyl group, a carboxyl group, or a formyl group, a 2-propynyl group, a 3-hydroxypropyl group, or a 2-hydroxypropyl group, and R⁴ represents a phenyl group or a phenyl group substituted with a carboxyl group].

4. A triterpene derivative represented by a general formula (I) or a pharmaceutically acceptable salt thereof: 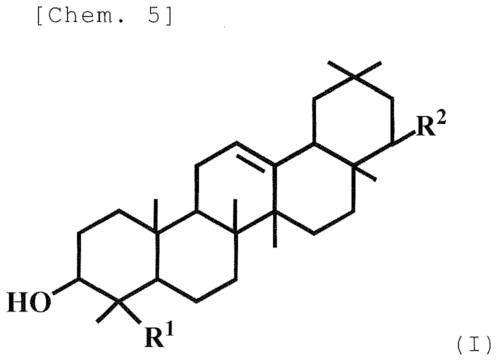 [in the formula (I), R¹ represents -CH₂OSO₃H, and R² represents a methoxy group].

5. A triterpene derivative represented by a general formula (I) or apharmaceuticallyacceptable salt thereof: 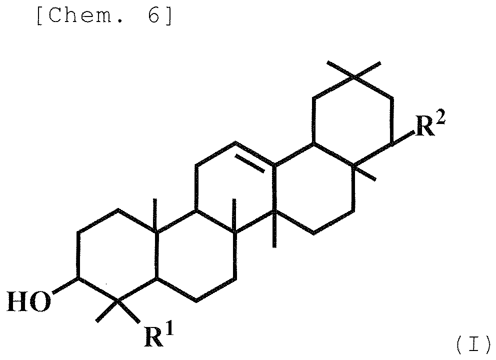 [in the formula (I), R¹ represents 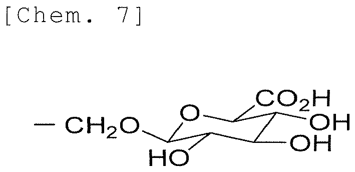 , and R² represents a methoxy group].

6. The triterpene derivative according to any one of claims 1 to 5 or a pharmaceutically acceptable salt thereof, wherein an IC50 value of inhibitory activity against hepatitis C virus is 20 µM or less.

7. An agent for preventing or treating chronic hepatitis C, comprising the triterpene derivative according to any one of claims 1 to 6 or a pharmaceutically acceptable salt thereof as an active ingredient.

8. The agent according to claim 7, comprising a pharmaceutically acceptable carrier.

9. The agent according to claim 7 or 8, further comprising an interferon in combination.

10. A method for preventing or treating chronic hepatitis C, comprising administering the agent according to any one of claim 7 to 9 in a prophylactically or therapeutically effective amount to a patient who needs prevention or treatment of chronic hepatitis C.

11. Use of the triterpene derivative or the pharmaceutically acceptable salt according to any one of claims 1 to 6, for producing an agent for preventing or treating chronic hepatitis C.

12. The use according to claim 11, wherein the agent for preventing or treating chronic hepatitis C is the agent according to any one of claims 7 to 9.

13. The triterpene derivative or the pharmaceutically acceptable salt according to any one of claims 1 to 6, for use in prevention or treatment of chronic hepatitis C.
